(19) Europäisches Patentamt
European Patent Office
Office européen des brevets

(11) **EP 4 082 547 B1**

(12) **EUROPEAN PATENT SPECIFICATION**

(45) Date of publication and mention
of the grant of the patent:
**04.02.2026 Bulletin 2026/06**

(21) Application number: **22178453.1**

(22) Date of filing: **02.12.2015**

(51) International Patent Classification (IPC):
*A61K 31/525* (2006.01)    *A61K 33/26* (2006.01)
*A61K 41/00* (2020.01)    *A61P 27/02* (2006.01)
*A61N 5/06* (2006.01)    *A61L 31/14* (2006.01)
*A61F 9/007* (2006.01)

(52) Cooperative Patent Classification (CPC):
(C-Sets available)
**A61K 41/00; A61F 9/0079; A61K 31/525;
A61K 33/26; A61L 31/14; A61N 5/062;
A61P 27/02;** A61L 2430/16    (Cont.)

(54) **SYSTEMS, METHODS, AND COMPOSITIONS FOR CROSS-LINKING TREATMENTS OF AN EYE**

SYSTEME, VERFAHREN UND ZUSAMMENSETZUNGEN FÜR BEHANDLUNGEN ZUR VERNETZUNG EINES AUGES

SYSTÈMES, PROCÉDÉS ET COMPOSITIONS POUR DES TRAITEMENTS DE RÉTICULATION D'UN OEIL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.12.2014 US 201462086572 P**

(43) Date of publication of application:
**02.11.2022 Bulletin 2022/44**

(62) Document number(s) of the earlier application(s) in
accordance with Art. 76 EPC:
**15864372.6 / 3 226 871**

(73) Proprietor: **Avedro, INC.
Waltham, MA 02451 (US)**

(72) Inventors:
• **KAMAEV, Pavel
Lexington (US)**
• **FRIEDMAN, Marc
Needham (US)**
• **SHERR, Evan
Ashland (US)**
• **PETERSON, Sarah, M.
Byfield (US)**
• **MULLER, David
Boston (US)**

(74) Representative: **Mewburn Ellis LLP
Aurora Building
Counterslip
Bristol BS1 6BX (GB)**

(56) References cited:
**US-A1- 2013 310 732    US-A1- 2014 343 480**

• **MARC D. FRIEDMAN ET AL: "Advanced Corneal
Cross-Linking System with Fluorescence
Dosimetry", JOURNAL OF OPHTHALMOLOGY,
vol. 2012, 1 January 2012 (2012-01-01), US, pages
1 - 6, XP055480767, ISSN: 2090-004X, DOI:
10.1155/2012/303459**
• **PAVEL KAMAEV ET AL: "Photochemical Kinetics
of Corneal Cross-Linking with Riboflavin",
INVESTIGATIVE OPTHALMOLOGY & VISUAL
SCIENCE, vol. 53, no. 4, 30 April 2012
(2012-04-30), US, pages 2360, XP055248371,
ISSN: 1552-5783, DOI: 10.1167/iovs.11-9385**

EP 4 082 547 B1

(52) Cooperative Patent Classification (CPC): (Cont.)

C-Sets
**A61K 31/525, A61K 2300/00;
A61K 33/26, A61K 2300/00**

**Description**

CROSS REFERENCE TO RELATED APPLICATIONS

This application claims priority to U.S. Provisional Patent Application No. 62/086,572, filed December 2, 2014.

BACKGROUND

Field

The present disclosure pertains to a composition for use in a method for applying treatment to a cornea of an eye.

Description of Related Art

Cross-linking treatments may be employed to treat eyes suffering from disorders, such as keratoconus. In particular, keratoconus is a degenerative disorder of the eye in which structural changes within the cornea cause it to weaken and change to an abnormal conical shape. Cross-linking treatments can strengthen and stabilize areas weakened by keratoconus and prevent undesired shape changes.

Cross-linking treatments may also be employed after surgical procedures, such as Laser-Assisted in situ Keratomileusis (LASIK) surgery. For instance, a complication known as post-LASIK ectasia may occur due to the thinning and weakening of the cornea caused by LASIK surgery. In post-LASIK ectasia, the cornea experiences progressive steepening (bulging). Accordingly, cross-linking treatments can strengthen and stabilize the structure of the cornea after LASIK surgery and prevent post-LASIK ectasia.

US 2014/0343480 A1 (KAMAEV PAVEL et al.) describes systems, methods and compositions for cross-linking. US 2013/310732 A1 (FOSCHINI FULVIO et al.) describes corneal delivery of cross-linking agents by iontophoresis for the treatment of keratoconus and related ophthalmic compositions.

BRIEF DESCRIPTION OF THE DRAWINGS

FIG. 1 illustrates an example system that delivers a cross-linking agent and photoactivating light to a cornea of an eye in order to generate cross-linking of corneal collagen, according to aspects of the present disclosure.

FIGS. 2A-B illustrate a diagram for photochemical kinetic reactions involving riboflavin and photoactivating light (e.g., ultraviolet A (UVA) light) applied during a corneal cross-linking treatment, according to aspects of the present disclosure.

FIG. 3 illustrates an example system employing a model of photochemical kinetic reactions according to aspects of the present disclosure.

FIG. 4 illustrates a diagram relating to formation of oxidants by electron transfer reactions and possible initiation of the polymerization by hydrogen abstraction.

FIG. 5 illustrates fluorescence of the corneal digests at 450 nm in relation to the non-cross-linked control (F/Fo) with different concentrations of Iron(II) in solution applied during cross-linking.

FIG. 6 illustrates fluorescence counts of papain digested corneal flaps treated with: (1) $dH_2O$; (2) $H_2O_2$; and (3) $H_2O_2$ and 0.1% Iron(II).

FIG. 7 illustrates fluorescence (relative to untreated controls) recorded at 450 nm for: (1) 0.1% riboflavin (continuous wave (CW)); (2) 0.1% riboflavin + 0.5 mM $FeSO_4$ (CW); (3) 0.1% riboflavin (pulsed wave (PW) + $O_2$); and (4) 0.1% riboflavin + 0.5 mM $FeSO_4$ (PW + $O_2$).

FIG. 8 illustrates average force vs. displacement of each corneal flap measured by tensiometry for: 0.1% riboflavin (CW) (2) and 0.1% riboflavin + 0.5 mM $FeSO_4$ (CW) (3), relative to controls (1).

FIGS. 9-10 illustrate, for repeated experiments, average force vs. displacement of each corneal flap measured by tensiometry for 0.1% riboflavin (PW + $O_2$) (2) and 0.1% riboflavin + 0.5 mM $FeSO_4$ (PW + $O_2$) (3), relative to controls (1).

FIG. 11 illustrates a mechanism for the formation of 2,3-butanedione from riboflavin and singlet oxygen.

FIG. 12 illustrates displacement-force diagrams for corneal flaps treated with 1% solution of 2,3-butanedione (BD) in $dH_2O$ without ultraviolet (UV) light (left panel) and with UV light (right panel) (365 nm, 30 mW for 4 min).

FIG. 13 illustrates fluorescence (relative to the untreated controls, Fo) recorded at 450 nm from the papain digested 200 μm-thick corneal flaps, treated with 1% solution of BD with and without UV light.

FIG. 14 illustrates displacement-force diagrams for corneal flaps treated with UV light (365 nm, 30 mW for 4 min) and:

0.1% solution of riboflavin in $dH_2O$ (1); 0.1% BD in $dH_2O$ (4); and mixture of 0.1% riboflavin and 0.1% BD in $dH_2O$ (3), relative to controls (1).

FIG. 15 illustrates fluorescence (relative to the untreated controls, Fo) recorded at 450 nm from the papain digested 200 $\mu$m-thick corneal flaps, treated with 30 mW UVA for 4 min and: 0.1% solution of riboflavin in $dH_2O$; 0.1% BD in $dH_2O$; and mixture of 0.1% riboflavin and 0.1% BD in $dH_2O$.

FIG. 16 illustrates folic acid (FA).

FIG. 17 illustrates pterine-6-carboxylic acid (PCA), a photoproduct of FA.

FIG. 18 illustrates the absorption spectrum of 0.001% FA in PBS.

FIG. 19 illustrates fluorescence spectrum of 0.001% FA in PBS (excitation 360 nm).

FIG. 20 illustrates absorbance of FA in phosphate buffer at 360 nm.

FIG. 21 illustrates displacement vs. force curves for corneal samples: (1) not exposed to UV light; (2) 0.1% riboflavin, exposed to UV light; (3) 0.1% FA, exposed to UV light; and (4) mixture of 0.1% riboflavin and 0.1% FA, exposed to UV light, with UV exposure of 365 nm, 30 $mW/cm^2$, pulsed 1 second on: 1 second off for 8 minutes total, with oxygen ambience over the cornea.

FIG. 22 illustrates fluorescence of the corneal samples after digestion with papain (excitation 360 nm): (1) not exposed to UV light; (2) 0.1% riboflavin, exposed to UV light; (3) 0.1% FA, exposed to UV light; and (4) mixture of 0.1% riboflavin and 0.1% FA, exposed to UV light, with UV exposure at 365 nm, 30 $mW/cm^2$, pulsed 1 second on: 1 second off for 8 minutes total, with oxygen ambience over the cornea.

FIG. 23 illustrates displacement vs. force curves for corneal samples (thickness 300 um, 3 samples in each group): (1) controls unexposed to UV light; (2) 0.1% FA in a buffer, exposed UV light; (3) 0.1% riboflavin in buffer saline solution, exposed to UV light; and (4) mixture of 0.1% FA in 0.1% riboflavin in buffer saline solution, exposed to UV light, UV exposure at 365 nm, 30 $mW/cm^2$, pulsed 1 second on: 1 second off for 8 minutes total, with oxygen ambience over the cornea.

FIG. 24 illustrates biaxial extensiometry of 200 um thick corneal flaps, soaked with 0.4% Olaquindox in PBS with irradiation for 4 min with 30 mW/cm2 (CW) (2) and irradiation for 8 minutes (1 second on:1 second off) with 30 mW/cm2 of pulsed UVA light and $O_2$ (3), relative to controls (1).

FIG. 25 illustrates relative fluorescence recorded at 450 nm of the cross-linked flaps with 0.4% Olaquindox in PBS: (1) non-irradiated control; (2) irradiation for 4 min with 30 $mW/cm^2$ continuously (CW); and (3): irradiation for 8 minutes (1 second on:1 second off) with 30 mW/cm2 of pulsed UVA light and $O_2$.

FIG. 26 illustrates alkaline hydrolysis of riboflavin (A) into 1,2-dihydro-6,7-dimethyl-2-keto-1-D-ribityl-3-quinoxaline-carboxylic acid (B).

FIG 27 illustrates UV/Vis spectra of the 0.1% riboflavin-5-phosphate in BBBS kept at 120 $^0$C for different amounts of time.

FIG. 28 illustrates the rate of hydrolysis of riboflavin at 120°C in BBBS as measured by absorbance at 450 nm (0.1% solution and 0.5% solution, $A_0$ is the absorbance before heating).

FIG. 29 illustrates UV/Vis spectra which is obtained from FIG. 27 by subtracting absorbance of the remaining riboflavin.

FIG. 30 illustrates spectral analysis of the hydrolyzed solution after 90 min at 120°C (absorbance of residual riboflavin was subtracted from the analyzed spectrum).

FIG. 31 illustrates change in the absorbance of the different peaks during the time of hydrolysis.

FIG. 32 illustrates the synthesis of the sodium salt of 1,2-Dihydro-6,7-dimethyl-2-keto-1-D-ribityl-3-quinoxalinecarboxylic acid monohydrate 2, an early stage alkaline degradation product of riboflavin.

FIG. 33 illustrates NMR spectrum of the synthesized riboflavin degradation product 2.

FIG. 34 illustrates monophosphorylated riboflavin (5-FMN) in buffered blood bank saline without thermal treatment.

FIG. 35 illustrates 5- FMN in buffered blood bank saline after 1 hour of thermal treatment.

FIG. 36 illustrates 5-FMN in buffered blood bank saline after 2 hours of thermal treatment.

FIG. 37 illustrates 5-FMN in buffered blood bank saline after 3 hours of thermal treatment.

FIG. 38 illustrates 5-FMN in buffered blood bank saline after 4 hours of thermal treatment.

FIG. 39 illustrates HPLC trace of the synthesized riboflavin degradation product 2.

FIG. 40 illustrates absorption spectra of thermally heated (red line) and not heated (blue line) riboflavin solutions (recorded in a quartz cuvette with 200 $\mu$m optical path).

FIG. 41 illustrates the Difference between two spectra in FIG. 34.

FIG. 42 illustrates fluorescence of the digested corneas: non-cross-linked control (black line), cross-linked with 0.1% riboflavin which was not heated (blue line), cross-linked with thermally treated riboflavin solution (red line).

FIG. 43 illustrates relative fluorescence of the cross-linked corneal samples: red - using thermally treated riboflavin solution, blue - using not heated 0.1% riboflavin solution.

FIG. 44 illustrates sodium salt of 1,2-Dihydro-6,7-dimethyl-2-keto-1-D-ribityl-3-quinoxalinecarboxylic acid monohydrate.

FIG. 45 illustrates absorbance spectrum of 0.001% solution of sodium salt of 1,2-Dihydro-6,7-dimethyl-2-keto-1-D-ribityl-3-quinoxalinecarboxylic acid monohydrate in BBBS (quartz, 1 cm light path)

FIG. 46 illustrates UV absorbance of sodium salt of 1,2-Dihydro-6,7-dimethyl-2-keto-1-D-ribityl-3-quinoxalinecarboxylic acid monohydrate at 360 nm (solution in BBBS, quartz cuvette with 1 cm light path).

FIG. 47 illustrates fluorescence of sodium salt of 1,2-Dihydro-6,7-dimethyl-2-keto-1-D-ribityl-3-quinoxalinecarboxylic acid monohydrate in BBBS solutions (excitation 360 nm).

FIG. 48 illustrates fluorescence of the digested corneas: non-cross-linked control (black line), cross-linked with 0.1% riboflavin in BBBS (red line), cross-linked with 0.1% sodium salt of 1,2-Dihydro-6,7-dimethyl-2-keto-1-D-ribityl-3-quinoxalinecarboxylic acid monohydrate (blue line).

FIG. 49 illustrates fluorescence of the digested corneal flaps: non-cross-linked control (black line), cross-linked with 0.1% riboflavin in BBBS (red line), cross-linked with 0.1% sodium salt of 1,2-Dihydro-6,7-dimethyl-2-keto-1-D-ribityl-3-quinoxalinecarboxylic acid monohydrate (blue line).

FIG. 50 illustrates 3-hydroxy-2-quinoxalinecarboxylic acid.

FIG. 51 illustrates absorbance spectra of 3-hydroxy-2-quinoxalinecarboxylic acid.

FIG. 52 illustrates fluorescence of 3-hydroxy-2-quinoxalinecarboxylic acid's solutions with different concentrations in BBBS, recorded with excitation of 360 nm.

FIG. 53 illustrates fluorescence of the papain digested corneal flaps (200 $\mu$m thick) cross-linked with 0.17% (red lines) and 0.017% (blue lines) solutions of 3-hydroxy-2-quinoxalinecarboxylic acid in BBBS (no epithelium, 20 min soaking time, 30 mW/cm$^2$ for 4 min), relative to non-cross-linked controls (black lines).

FIG. 54 illustrates tensiometry plots of 200- $\mu$m thick corneal flaps irradiated at 30 mW/cm2 for 4 min, preliminary saturated for 20 min with 0.17% riboflavin (red lines) and 0.17% 3-hydroxy-2-quinoxalinecarboxylic acid (3H2QXCA, green lines)relative to non-cross-linked controls (black lines).

FIG. 55 illustrates relative fluorescence of the papain digested corneal flaps (200 $\mu$m thick) cross-linked with 0.1% riboflavin (blue bar, solution 2) and 0.1% riboflavin containing 0.02% solution of 3-hydroxy-2-quinoxalinecarboxylic acid in BBBS (red bar, solution 1) (no epithelium, 20 min soaking time, 30 mW/cm$^2$ for 4 min), where $F_0$ - fluorescence of a non-cross-linked flap.

FIG. 56 illustrates 4-methyl-3-oxo-3,4-dihydro-2-quinoxaline carboxylic acid.

FIG. 57 illustrates absorbance spectrum of 0.01 mg/ml solution of 4-methyl-3-oxo-3,4-dihydro-2-quinoxaline carboxylic acid in BBBS (quartz, 1 cm light path).

FIG. 58 illustrates fluorescence of 4-methyl-3-oxo-3,4-dihydro-2-quinoxaline carboxylic acid in BBBS solutions (excitation 360 nm).

FIG. 59 illustrates fluorescence of the papain-digested corneal flaps: non-cross-linked control (black line), cross-linked with 0.1 mg/ml (red line) and 1 mg/ml (green line) solutions of 4-methyl-3-oxo-3,4-dihydro-2-quinoxaline carboxylic acid in BBBS, where corneas were de-epithelialized, soaked with the solution of the cross-linker for 20 min and then irradiated for 4 min with 30 mW/cm$^2$ UVA light (360 nm).

FIG. 60 illustrates quinoxaline (also called benzopyrazine) as a heterocyclic compound containing a ring complex made up of benzene ring and a pyrazine ring.

FIG. 61 illustrate quinoxaline-2,3-dithione cyclic dithio-carbonate (Morestan) and trithiocarbonate (Eradox).

FIG. 62 illustrates Methotrexate.

FIG. 63 illustrates menadione (vitamin $K_3$),

FIG. 64 illustrates the relative fluorescence of the cross-linked flaps with 0.1% riboflavin (buffer saline solution) versus 0.1% riboflavin (buffer saline solution) + 0.25 mM Iron + 0.5mM Citrate Buffer.

FIG. 65 illustrates the relative fluorescence of the cross-linked flaps with 0.1% riboflavin (buffer saline solution) versus 0.1% riboflavin (buffer saline solution) + 0.5mM Iron + 0.25mM Citrate Buffer.

While the present disclosure is susceptible to various modifications and alternative forms, a specific embodiment thereof has been shown by way of example in the drawings and will herein be described in detail. It should be understood, however, that it is not intended to limit the present disclosure to the particular forms disclosed, but on the contrary, the intention is to cover all modifications, equivalents, and alternatives.

SUMMARY

Aspects of the present disclosure provide systems, methods, and compositions that generate cross-linking activity for treatment of eye disorders. Various agents, additives, buffers, etc., for cross-linking treatments are identified, for example, in studies disclosed herein. The characteristics of the various agents, additives, buffers, etc., may be employed in formulations with a cross-linking agent to enhance cross-linking treatments.

Described herein is a composition for applying treatment to a cornea of an eye includes a cross-linking agent that generates cross-linking activity in the cornea in response to exposure to a photo-activating light. The composition also includes an iron additive and citrate buffer. In some cases, the cross-linking agent may include riboflavin. In other cases, the iron additive may include $FeSO_4$. In further cases, the iron additive may be dissolved in the citrate buffer. In yet other cases, the photo-activating light may be ultraviolet light.

The present invention provides a composition for use in a method for applying treatment to a cornea of an eye, wherein the method includes applying a composition to the cornea. The composition includes a cross-linking agent that generates cross-linking activity in the cornea in response to exposure to a photo-activating light. The cross-linking agent includes at least one of riboflavin, 2,3-butanedione, folic acid, quinolone, dibucaine, or a quinoxaline. The composition also includes an iron additive; and citrate buffer. The method also includes applying photo-activating light to the cornea to generate cross-linking activity in the cornea. In some cases, the cross-linking agent may include riboflavin. In some embodiments, the iron additive may include $FeSO_4$. In further embodiments, the iron additive may be dissolved in the citrate buffer. In some embodiments, the photo-activating light may be ultraviolet light. In some embodiments, the photo-activating light may be pulsed. In other embodiments, the photo-activating light may be continuous wave. The method may further include applying a selected concentration of oxygen to the eye, where the selected concentration is greater than a concentration of oxygen in atmosphere. In some embodiments, the cornea is weakened due to keratoconus. In other embodiments, the cornea is weakened due to Laser-Assisted in situ Keratomileusis (LASIK) ectasia.

DESCRIPTION

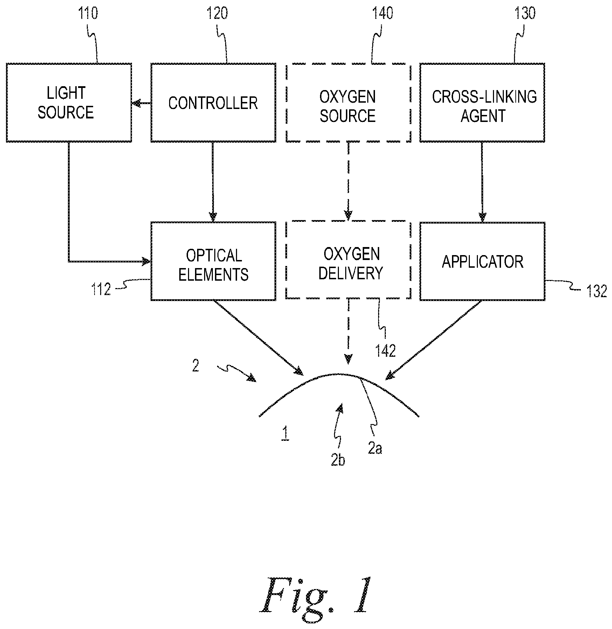

FIG. 1 illustrates an example treatment system 100 for generating cross-linking of collagen in a cornea 2 of an eye 1. The treatment system 100 includes an applicator 132 for applying a cross-linking agent 130 to the cornea 2. As described herein, the applicator 132 may be an eye dropper, syringe, or the like that applies the photosensitizer 130 as drops to the cornea 2. The cross-linking agent 130 may be provided in a formulation that allows the cross-linking agent 130 to pass through the corneal epithelium 2a and to underlying regions in the corneal stroma 2b. Alternatively, the corneal epithelium 2a may be removed or otherwise incised to allow the cross-linking agent 130 to be applied more directly to the underlying tissue.

The treatment system 100 includes a light source 110 and optical elements 112 for directing light to the cornea 2. The light causes photoactivation of the cross-linking agent 130 to generate cross-linking activity in the cornea 2. For example, the cross-linking agent may include riboflavin and the photoactivating light may be ultraviolet A (UVA) (e.g., 365 nm) light. Alternatively, the photoactivating light may have another wavelength, such as a visible wavelength (e.g., 452 nm). As described further below, corneal cross-linking improves corneal strength by creating chemical bonds within the corneal tissue according to a system of photochemical kinetic reactions. For instance, riboflavin and the photoactivating light are applied to stabilize and/or strengthen corneal tissue to address diseases such as keratoconus or post-LASIK ectasia. Additionally, as described further below, various agents, additives, buffers, etc., may be employed in formulations with the cross-linking agent to enhance cross-linking treatments.

The treatment system 100 includes one or more controllers 120 that control aspects of the system 100, including the light source 110 and/or the optical elements 112. In an implementation, the cornea 2 can be more broadly treated with the cross-linking agent 130 (e.g., with an eye dropper, syringe, etc.), and the photoactivating light from the light source 110 can be selectively directed to regions of the treated cornea 2 according to a particular pattern.

The optical elements 112 may include one or more mirrors or lenses for directing and focusing the photoactivating light emitted by the light source 110 to a particular pattern on the cornea 2. The optical elements 112 may further include filters for partially blocking wavelengths of light emitted by the light source 110 and for selecting particular wavelengths of light to be directed to the cornea 2 for activating the cross-linking agent 130. In addition, the optical elements 112 may include one or more beam splitters for dividing a beam of light emitted by the light source 110, and may include one or more heat sinks for absorbing light emitted by the light source 110. The optical elements 112 may also accurately and precisely focus the photo-activating light to particular focal planes within the cornea 2, e.g., at a particular depths in the underlying region 2b where cross-linking activity is desired.

Moreover, specific regimes of the photoactivating light can be modulated to achieve a desired degree of cross-linking in the selected regions of the cornea 2. The one or more controllers 120 may be used to control the operation of the light source 110 and/or the optical elements 112 to precisely deliver the photoactivating light according to any combination of: wavelength, bandwidth, intensity, power, location, depth of penetration, and/or duration of treatment (the duration of the exposure cycle, the dark cycle, and the ratio of the exposure cycle to the dark cycle duration).

The parameters for photoactivation of the cross-linking agent 130 can be adjusted, for example, to reduce the amount of time required to achieve the desired cross-linking. In an example implementation, the time can be reduced from minutes to seconds. While some configurations may apply the photoactivating light at an irradiance of 5 mW/cm$^2$, larger irradiance of the photoactivating light, e.g., multiples of 5 mW/cm$^2$, can be applied to reduce the time required to achieve the desired cross-linking. The total dose of energy absorbed in the cornea 2 can be described as an effective dose, which is an amount of energy absorbed through an area of the corneal epithelium 2a. For example the effective dose for a region of the corneal surface 2A can be, for example, 5 J/cm$^2$, or as high as 20 J/cm$^2$ or 30 J/cm$^2$. The effective dose described can be delivered from a single application of energy, or from repeated applications of energy.

The optical elements 112 of the treatment system 100 may include a digital micro-mirror device (DMD) to modulate the application of photoactivating light spatially and temporally. Using DMD technology, the photoactivating light from the light source 110 is projected in a precise spatial pattern that is created by microscopically small mirrors laid out in a matrix on a semiconductor chip. Each mirror represents one or more pixels in the pattern of projected light. With the DMD one can perform topography guided cross-linking. The control of the DMD according to topography may employ several different spatial and temporal irradiance and dose profiles. These spatial and temporal dose profiles may be created using continuous wave illumination but may also be modulated via pulsed illumination by pulsing the illumination source under varying frequency and duty cycle regimes as described above. Alternatively, the DMD can modulate different frequencies and duty cycles on a pixel by pixel basis to give ultimate flexibility using continuous wave illumination. Or alternatively, both pulsed illumination and modulated DMD frequency and duty cycle combinations may be combined. This allows for specific amounts of spatially determined corneal cross-linking. This spatially determined cross-linking may be combined with dosimetry, interferometry, optical coherence tomography (OCT), corneal topography, etc., for pre-treatment planning and/or real-time monitoring and modulation of corneal cross-linking during treatment. Additionally, pre-clinical patient information may be combined with finite element biomechanical computer modeling to create patient specific pre-treatment plans.

To control aspects of the delivery of the photoactivating light, aspects of multiphoton excitation microscopy may be employed. In particular, rather than delivering a single photon of a particular wavelength to the cornea 2, the treatment system 100 may deliver multiple photons of longer wavelengths, i.e., lower energy, that combine to initiate the cross-linking. Advantageously, longer wavelengths are scattered within the cornea 2 to a lesser degree than shorter wavelengths, which allows longer wavelengths of light to penetrate the cornea 2 more efficiently than shorter wavelength light. Shielding effects of incident irradiation at deeper depths within the cornea are also reduced over conventional short wavelength illumination since the absorption of the light by the photosensitizer is much less at the longer wavelengths. This allows for enhanced control over depth specific cross-linking. For example, , two photons may be employed, where each photon carries approximately half the energy necessary to excite the molecules in the cross-linking agent 130 to generate the photochemical kinetic reactions described further below. When a cross-linking agent molecule simultaneously absorbs both photons, it absorbs enough energy to release reactive radicals in the corneal tissue. Lower energy photons may also be utilized such that a cross-linking agent molecule must simultaneously absorb, for example, three, four, or five, photons to release a reactive radical. The probability of the near-simultaneous absorption of multiple photons is low, so a high flux of excitation photons may be required, and the high flux may be delivered through a femtosecond laser.

A large number of conditions and parameters affect the cross-linking of corneal collagen with the cross-linking agent 130. For example, when the cross-linking agent 130 is riboflavin and the photoactivating light is UVA light, the irradiance and the dose both affect the amount and the rate of cross-linking. The UVA light may be applied continuously (continuous wave (CW)) or as pulsed light, and this selection has an effect on the amount, the rate, and the extent of cross-linking.

If the UVA light is applied as pulsed light, the duration of the exposure cycle, the dark cycle, and the ratio of the exposure cycle to the dark cycle duration have an effect on the resulting corneal stiffening. Pulsed light illumination can be used to create greater or lesser stiffening of corneal tissue than may be achieved with continuous wave illumination for the same amount or dose of energy delivered. Light pulses of suitable length and frequency may be used to achieve more optimal chemical amplification. For pulsed light treatment, the on/off duty cycle may be between approximately 1000/1 to approximately 1/1000; the irradiance may be between approximately 1 mW/cm$^2$ to approximately 1000 mW/cm$^2$ average irradiance, and the pulse rate may be between approximately 0.01 HZ to approximately 1000 Hz or between approximately 1000 Hz to approximately 100,000 Hz.

The treatment system 100 may generate pulsed light by employing a DMD, electronically turning the light source 110 on and off, and/or using a mechanical or optoelectronic (e.g., Pockels cells) shutter or mechanical chopper or rotating aperture. Because of the pixel specific modulation capabilities of the DMD and the subsequent stiffness impartment based on the modulated frequency, duty cycle, irradiance and dose delivered to the cornea, complex biomechanical stiffness patterns may be imparted to the cornea to allow for various amounts of refractive correction. These refractive corrections, for example, may involve combinations of myopia, hyperopia, astigmatism, irregular astigmatism, presbyopia and complex corneal refractive surface corrections because of ophthalmic conditions such as keratoconus, pellucid marginal disease, post-lasik ectasia, and other conditions of corneal biomechanical alteration/degeneration, etc. A specific advantage of the DMD system and method is that it allows for randomized asynchronous pulsed topographic patterning, creating a non-periodic and uniformly appearing illumination which eliminates the possibility for triggering photosensitive epileptic seizures or flicker vertigo for pulsed frequencies between 2 Hz and 84 Hz.

Although stepwise on/off pulsed light functions may be employed, it is understood that other functions for applying light to the cornea may be employed to achieve similar effects. For example, light may be applied to the cornea according to a sinusoidal function, sawtooth function, or other complex functions or curves, or any combination of functions or curves. Indeed, it is understood that the function may be substantially stepwise where there may be more gradual transitions between on/off values. In addition, it is understood that irradiance does not have to decrease down to a value of zero during the off cycle, and may be above zero during the off cycle. Desired effects may be achieved by applying light to the cornea according to a curve varying irradiance between two or more values.

Examples of systems and methods for delivering photoactivating light are described, for example, in U.S. Patent Application Publication No. 2011/0237999, filed March 18, 2011 and titled "Systems and Methods for Applying and Monitoring Eye Therapy," U.S. Patent Application Publication No. 2012/0215155, filed April 3, 2012 and titled "Systems and Methods for Applying and Monitoring Eye Therapy," and U.S. Patent Application Publication No. 2013/0245536, filed March 15, 2013 and titled "Systems and Methods for Corneal Cross-Linking with Pulsed Light,".

The addition of oxygen also affects the amount of corneal stiffening. In human tissue, $O_2$ content is very low compared to the atmosphere. The rate of cross-linking in the cornea, however, is related to the concentration of $O_2$ when it is irradiated with photoactivating light. Therefore, it may be advantageous to increase or decrease the concentration of $O_2$ actively during irradiation to control the rate of cross-linking until a desired amount of cross-linking is achieved. Oxygen may be applied during the cross-linking treatments in a number of different ways. One approach involves supersaturating the riboflavin with $O_2$. Thus, when the riboflavin is applied to the eye, a higher concentration of $O_2$ is delivered directly into the cornea with the riboflavin and affects the reactions involving $O_2$ when the riboflavin is exposed to the photoactivating light. According to another approach, a steady state of $O_2$ (at a selected concentration) may be maintained at the surface of the cornea to expose the cornea to a selected amount of $O_2$ and cause $O_2$ to enter the cornea. As shown in FIG. 1, for instance, the treatment system 100 also includes an oxygen source 140 and an oxygen delivery device 142 that optionally delivers oxygen at a selected concentration to the cornea 2. Example systems and methods for applying oxygen during cross-linking treatments are described, for example, in U.S. Patent No. 8,574,277, filed October 21, 2010 and titled "Eye Therapy," U.S. Patent Application Publication No. 2013/0060187, filed October 31, 2012 and titled "Systems and Methods for Corneal Cross-Linking with Pulsed Light,".

When riboflavin absorbs radiant energy, especially light, it undergoes photoactivation. There are two photochemical kinetic pathways for riboflavin photoactivation, Type I and Type II. Some of the reactions involved in both the Type I and Type II mechanisms are as follows:

**Common reactions:**

$$Rf \rightarrow Rf_1^*, \qquad I; \qquad (r1)$$
$$Rf_1^* \rightarrow Rf, \qquad \kappa 1; \qquad (r2)$$
$$Rf_1^* \rightarrow Rf_3^*, \qquad \kappa 2; \qquad (r3)$$

**Type I reactions:**

$$Rf_3^* + DH \rightarrow RfH^{\cdot} + D^{\cdot}, \qquad \kappa 3; \qquad (r4)$$
$$2RfH^{\cdot} \rightarrow Rf + RfH_2, \qquad \kappa 4; \qquad (r5)$$

**Type II reactions:**

$$DH + O_2^1 \rightarrow D_{ox} \qquad \kappa 5; \qquad (r6)$$
$$Rf_3^* + O_2 \rightarrow Rf + O_2^1, \qquad \kappa 6; \qquad (r7)$$
$$D_{ox} + DH \rightarrow D\text{-}D, \qquad \kappa 7; \qquad \mathbf{CXL} \qquad (r8)$$

In the reactions described herein, Rf represents riboflavin in the ground state. $Rf^*_1$ represents riboflavin in the excited singlet state. $Rf^*_3$ represents riboflavin in a triplet excited state. $Rf^{\cdot -}$ is the reduced radical anion form of riboflavin. $RfH^{\cdot}$ is the radical form of riboflavin. $RfH_2$ is the reduced form of riboflavin. DH is the substrate. $DH^{\cdot +}$ is the intermediate radical cation. $D^{\cdot}$ is the radical. $D_{ox}$ is the oxidized form of the substrate.

Riboflavin is excited into its triplet excited state $Rf^*_3$ as shown in reactions (r1) to (r3). From the triplet excited state $Rf^*_3$, the riboflavin reacts further, generally according to Type I or Type II mechanisms. In the Type I mechanism, the substrate reacts with the excited state riboflavin to generate radicals or radical ions, respectively, by hydrogen atoms or electron transfer. In Type II mechanism, the excited state riboflavin reacts with oxygen to form singlet molecular oxygen. The singlet molecular oxygen then acts on tissue to produce additional cross-linked bonds.

Oxygen concentration in the cornea is modulated by UVA irradiance and temperature and quickly decreases at the beginning of UVA exposure. Utilizing pulsed light of a specific duty cycle, frequency, and irradiance, input from both Type I and Type II photochemical kinetic mechanisms can be employed to achieve a greater amount of photochemical efficiency. Moreover, utilizing pulsed light allows regulating the rate of reactions involving riboflavin. The rate of reactions may either be increased or decreased, as needed, by regulating, one of the parameters such as the irradiance, the dose, the on/off duty cycle, riboflavin concentration, soak time, and others. Moreover, additional ingredients that affect the reaction and cross-linking rates may be added to the cornea.

If UVA radiation is stopped shortly after oxygen depletion, oxygen concentrations start to increase (replenish). Excess oxygen may be detrimental in the corneal cross-linking process because oxygen is able to inhibit free radical photopolymerization reactions by interacting with radical species to form chain-terminating peroxide molecules. The pulse rate, irradiance, dose, and other parameters can be adjusted to achieve a more optimal oxygen regeneration rate. Calculating and adjusting the oxygen regeneration rate is another example of adjusting the reaction parameters to achieve a desired amount of corneal stiffening.

Oxygen content may be depleted throughout the cornea, by various chemical reactions, except for the very thin corneal layer where oxygen diffusion is able to keep up with the kinetics of the reactions. This diffusion-controlled zone will gradually move deeper into the cornea as the reaction ability of the substrate to uptake oxygen decreases.

Riboflavin is reduced (deactivated) reversibly or irreversibly and/or photo-degraded to a greater extent as irradiance increases. Photon optimization can be achieved by allowing reduced riboflavin to return to ground state riboflavin in Type I reactions. The rate of return of reduced riboflavin to ground state in Type I reactions is determined by a number of factors. These factors include, but are not limited to, on/off duty cycle of pulsed light treatment, pulse rate frequency, irradiance, and dose. Moreover, the riboflavin concentration, soak time, and addition of other agents, including oxidizers, affect the rate of oxygen uptake. These and other parameters, including duty cycle, pulse rate frequency, irradiance, and dose can be selected to achieve more optimal photon efficiency and make efficient use of both Type I as well as Type II photochemical kinetic mechanisms for riboflavin photosensitization. Moreover, these parameters can be selected in such a way as to achieve a more optimal chemical amplification effect.

In addition to the photochemical kinetic reactions (r1)-(r8) above, however, the present inventors have identified the following photochemical kinetic reactions (r9)-(r26) that also occur during riboflavin photoactivation:

| | | | |
|---|---|---|---|
| $Rf_3^* \rightarrow Rf,$ | $\kappa 8;$ | | (r9) |
| $Rf_3^* + Rf \rightarrow 2RfH^{\cdot},$ | $\kappa 9;$ | | (r10) |
| $RfH_2 + O_2 \rightarrow RfH^{\cdot} + H^+ + O_2^-,$ | $\kappa 10;$ | | (r11) |
| $RfH^{\cdot} + O_2 \rightarrow Rf + H^+ + O_2^-,$ | $\kappa 11;$ | | (r12) |
| $2\,RfH_2 + O_2^- \rightarrow 2\,RfH^{\cdot} + H_2O_2,$ | $\kappa 12:$ | | (r13) |
| $2\,RfH^{\cdot} + O_2^- \rightarrow 2\,Rf + H_2O_2,$ | $\kappa 13;$ | | (r14) |
| $RfH + H_2O_2 \rightarrow OH^{\cdot} + Rf + H_2O,$ | $\kappa 14;$ | | (r15) |
| $OH^{\cdot} + DH \rightarrow D^{\cdot} + H_2O,$ | $\kappa 15;$ | | (r16) |
| $D^{\cdot} + D^{\cdot} \rightarrow D\text{-}D,\quad O_2^1 \rightarrow O_2,$ | $\kappa 16;\ k18;$ | **CXL** | (r17) (r18) |
| $D^{\cdot} + RfH_2 \rightarrow RfH^{\cdot} + DH,$ | $\kappa 19;$ | | (r19) |

$$Rf + Rf \underset{\kappa_a^-}{\overset{\kappa_a^+}{\rightleftharpoons}} A_1, \qquad \kappa_a = \kappa_a^+ / \kappa_a^- \qquad \text{(r20)}$$

$$RfH_2 + RfH_2 \underset{\kappa_a^-}{\overset{\kappa_a^+}{\rightleftharpoons}} A_2, \qquad \kappa_a = \kappa_a^+ / \kappa_a^- \qquad \text{(r21)}$$

$$Rf + RfH_2 \underset{\kappa_b^-}{\overset{\kappa_b^+}{\rightleftharpoons}} A_3, \qquad \kappa_b = \kappa_b^+ / \kappa_b^- \qquad \text{(r22)}$$

| | | |
|---|---|---|
| $Rf_1^* + A \rightarrow Rf + A,$ | $\kappa_{1a}$ | (r23) |
| $Rf_3^* + A \rightarrow Rf + A,$ | $\kappa_{3a}$ | (r24) |
| $2\,O_2^- \rightarrow O_2 + H_2O_2,$ | $\kappa_{12}$ | (r25) |
| $OH^{\circ} + CXL \rightarrow \text{inert products},$ | $\kappa_{OH}$ | (r26) |

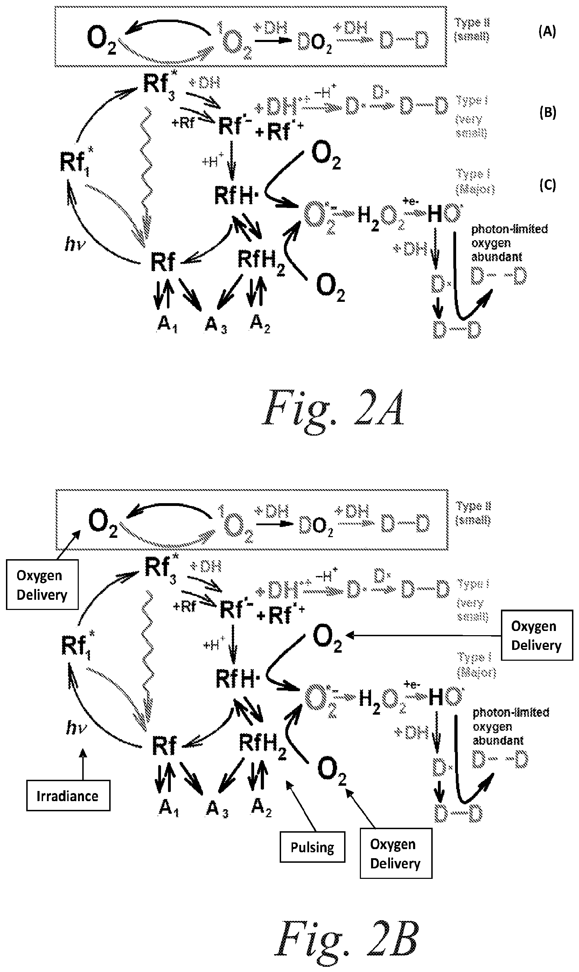

FIG. 2A illustrates a diagram for the photochemical kinetic reactions provided in reactions (r1) through (r26) above. The diagram summarizes photochemical transformations of riboflavin (Rf) under UVA photoactivating light and its interactions with various donors (DH) via electron transfer. As shown, cross-linking activity occurs: (A) through the presence of singlet oxygen in reactions (r6) through (r8) (Type II mechanism); (B) without using oxygen in reactions (r4) and (r17) (Type I mechanism); and (C) through the presence of peroxide ($H_2O_2$), superoxide ($O_2^-$), and hydroxyl radicals (˙OH) in reactions (r13) through (r17).

As shown in FIG. 2A, the present inventors have also determined that the cross-linking activity is generated to a greater degree from reactions involving peroxide, superoxide, and hydroxyl radicals. Cross-linking activity is generated to a lesser degree from reactions involving singlet oxygen and from non-oxygen reactions. Some models based on the reactions (r1)-(r26) may account for the level of cross-linking activity generated by the respective reactions. For instance, where singlet oxygen plays a smaller role in generating cross-linking activity, models may be simplified by treating the cross-linking activity resulting from singlet oxygen as a constant.

All the reactions start from $Rf_3^*$ as provided in reactions (r1)-(r3). The quenching of $Rf_3^*$ occurs through chemical reaction with ground state Rf in reaction (r10), and through deactivation by the interaction with water in reaction (r9).

As described above, excess oxygen may be detrimental in corneal cross-linking process. As shown in FIG. 2A, when the system becomes photon-limited and oxygen-abundant, cross-links can be broken from further reactions involving superoxide, peroxide, and hydroxyl radicals. Indeed, in some cases, excess oxygen may result in net destruction of cross-links versus generation of cross-links.

As described above, a large variety of factors affect the rate of the cross-linking reaction and the amount of biomechanical stiffness achieved due to cross-linking. A number of these factors are interrelated, such that changing one factor may have an unexpected effect on another factor. However, a more comprehensive model for understanding the relationship between different factors for cross-linking treatment is provided by the photochemical kinetic reactions (r1)-(r26) identified above. Accordingly, systems and methods can adjust various parameters for cross-linking treatment according to this photochemical kinetic cross-linking model, which provides a unified description of oxygen dynamics and cross-linking activity. The model can be employed to evaluate expected outcomes based on different combinations of treatment parameters and to identify the combination of treatment parameters that provides the desired result. The parameters, for example, may include, but is not limited to: the concentration(s) and/or soak times of the applied cross-linking agent; the dose(s), wavelength(s), irradiance(s), duration(s), and/or on/off duty cycle(s) of the photoactivating light; the oxygenation conditions in the tissue; and/or presence of additional agents and solutions.

A model based on aspects of the reactions (r1)-(r26) has been validated by at least five different methods of evaluating cross-linking activity:

- Oxygen depletion experiments
- Non-linear optical microscopy fluorescence experiments
- Fluorescence data based on papain digestion method experiments
- Corneal stromal demarcation line correlation experiments
- Brillouin microscopy experiements

These evaluations, for example, are described in PCT International Patent Application No. PCT/US15/57628, filed on October 27, 2015, and U.S. Provisional Patent Application No. 62/255,452, filed on November 14, 2015.

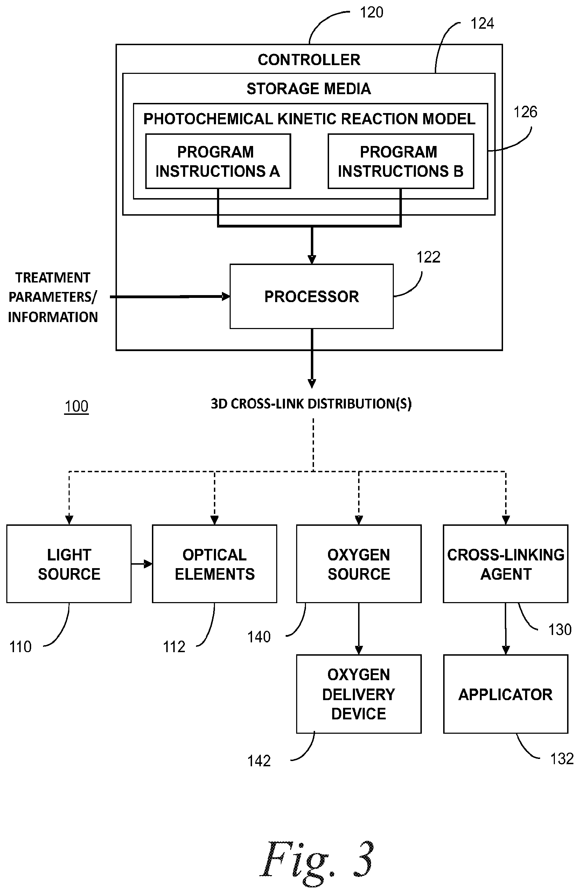

FIG. 3 illustrates the example system 100 employing a model based on the photochemical kinetic reactions (r1)-(r26) identified above. The controller 120 includes a processor 122 and computer-readable storage media 124. The storage media 124 stores program instructions for determining an amount of cross-linking when the photoactivating light from the light source 110 is delivered to a selected region of a cornea treated with a cross-linking agent. In particular, a photochemical kinetic model 126 based on the reactions (r1)-(r26) may include a first set of program instructions A for determining cross-linking resulting from reactions involving reactive oxygen species (ROS) including combinations of peroxides, superoxides, hydroxyl radicals, and/or singlet oxygen and a second set of program instructions B for determining cross-linking from reactions not involving oxygen. The controller 120 receives input relating to treatment parameters and/or other related information. The controller 120 can then execute the program instructions A and B to output information relating to three-dimensional cross-link distribution(s) for the selected region of the cornea based on the input. The three-dimensional cross-link distribution(s) may then be employed to determine how to control aspects of the light source 110, the optical elements 112, the cross-linking agent 130, the applicator 132, the oxygen source 140, and/or oxygen delivery device 142 in order to achieve a desired treatment in selected region of the cornea. (Of course, the system 100 shown in FIG. 3 and this process can be used for treatment of more than one selected region of the same cornea.)

According to one implementation, the three-dimensional cross-link distribution(s) may be evaluated to calculate a threshold depth corresponding to a healing response due to the cross-links and an effect of the reactive-oxygen species in the selected region of the cornea. Additionally or alternatively, the three-dimensional cross-link distribution(s) may be evaluated to calculate a biomechanical tissue stiffness threshold depth corresponding to a biomechanical tissue response in the selected region of the cornea. The information on the depth of the healing response and/or the biomechanical tissue stiffness in the cornea can be employed to determine how to control aspects of the light source 110, the optical elements 112, the cross-linking agent 130, the applicator 132, the oxygen source 140, and/or oxygen delivery device 142. Certain healing response and/or biomechanical tissue stiffness may be desired or not desired at certain depths of the cornea.

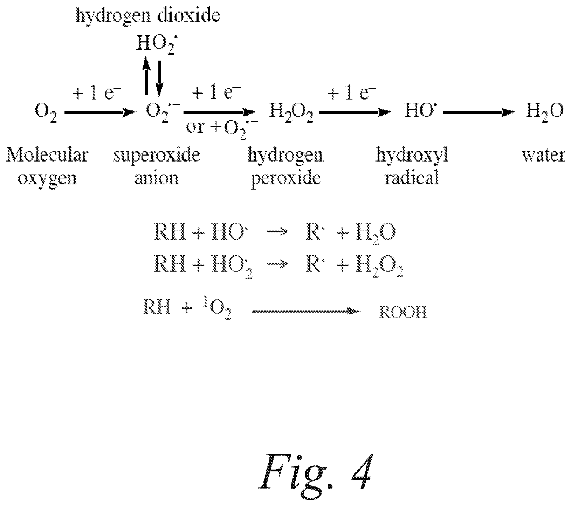

As described above, FIGS. 2A-B illustrate a diagram for the photochemical kinetic reactions involving riboflavin and photoactivating light (e.g., ultraviolet A (UVA) light) applied during a corneal cross-linking treatment. Not all the superoxide anions generated in the reactions shown in FIGS. 2A-B, however, are consumed in the overall reaction. Superoxide anions (in equilibrium with its conjugate acid) can produce hydrogen peroxide and subsequently hydroxyl radicals, as shown in the diagram of FIG. 4. In particular, FIG. 4 shows the formation of oxidants by electron transfer reactions and possible initiation of the polymerization by hydrogen abstraction. The overall picture of the action of different reactive oxygen species (ROS) in FIG. 4 on collagen is complex. Superoxide anions are not very reactive, but are able to degrade collagen. As opposite, hydroxyl radicals alone are able to induce protein aggregation. Hydroxyl radicals are considered to be initiators of polymerization not only for proteins. However, a mixture of the hydroxyl radicals with superoxide anions (with excess of the former) stimulates degradation of proteins.

FIG. 4 establishes that hydrogen peroxide is the immediate precursor of the hydroxyl radicals. To increase the concentration of hydroxyl radicals and accelerate collagen cross-linking, the decomposition of hydrogen peroxide may be accelerated. One way to do so involves employing Fenton's reaction:

$$H_2O_2 + Fe(II) \rightarrow OH^- + OH + Fe(III)$$

Concentration of the Fe(II) in solution is not too high because Fe(III) reacts with superoxide anion regenerating Fe(II):

$$Fe(III) + O_2^{\cdot -} \rightarrow O_2 + Fe(II)$$

Moreover, hydroxy-complexes of Fe(III), while irradiated with UV light photo-chemically reduces into Fe(II). Hydroxyl radicals generated during this photo-reduction is an additional bonus:

$$Fe(III)\text{-}OH + (UV\ light) \rightarrow Fe(II) + OH^{\cdot}$$

Copper ions can be used instead of iron, and it is a promising sign that cross-linking of collagen is observed under this condition.

The addition of traces of metals such as iron or copper to riboflavin formulations enhances corneal collagen cross-linking with UV light. Other metals that may mediate formation of reactive oxygen and nitrogen species include, for example: manganese, chromium, vanadium, aluminum, cobalt, mercury, cadmium, nickel, or arsenic.

In general, various agents, additives, buffers, etc., for cross-linking treatments are identified in the studies below. The characteristics of the various agents, additives, buffers, etc., may be employed in formulations with a cross-linking agent to enhance cross-linking treatments.

**Cross-linking with Ribolfavin and Iron(II)**

The following study conducted an investigation to establish how the presence of Iron(II) in riboflavin solution enhances collagen-related fluorescence indicative of cross-linking activity. The compositions used in this study do not fall within the scope of the invention.

A. Materials and Method

De-epithelialized eyes were soaked for 20 minutes with 0.1% riboflavin in dH$_2$O only, or 1 mM of FeSO$_4$ (Iron(II) Sulfate) in 0.1% riboflavin in dH$_2$O, in an incubator set at 37°C by using a rubber ring to hold the solution on top of the cornea. Corneas were pan-corneally irradiated with a top hat beam (3% root mean square) for 8 minutes (7.2 J total dose) in a cylinder filled with oxygen with 365-nm light source (pulsing 1 second on, 1 second off) (UV LED NCSU033B[T]; Nichia Co., Tokushima, Japan) at the chosen irradiance (30 mW/cm$^2$) which was measured with a power sensor (model PD-300-UV; Ophir, Inc., Jerusalem, Israel) at the corneal surface. Before the start of irradiation, oxygen's exposure was 2 minutes. Corneal flaps (approximately 200 μm thick) were excised from the eyes with aid of Intralase femtosecond laser (Abbot Medical Optics, Santa Ana, CA). The average thickness of the corneal flaps was calculated as a difference between the measurements before and after the excision from the eyes with an ultrasonic Pachymeter (DGH Technology, Exton, PA). The flaps were washed with distilled water two times, dried with filter paper, washed with dH$_2$O two times, and then dried in a vacuum until the weight change became less than 10% (Rotary vane vacuum pump RV3 A652-01-903, BOC Edwards, West Sussex, UK). Each flap was digested for 2.5 hours at 65°C with 2.5 units/ml of papain (from Papaya latex, Sigma) in 1 ml of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with BBBS and fluorescence of the solutions was measured with excitation of $\lambda$ex = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

This method was used because the non-enzymatic cross-link density in collagens was previously quantified with use of papain digest fluorescence. There is a linear relationship between fluorescence and increasing cross-linking activity.

B. Results/Conclusion

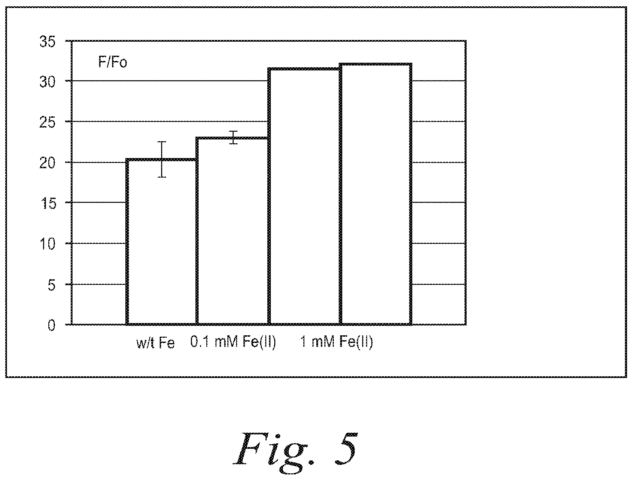

FIG. 5 illustrates the fluorescence of the corneal digests at 450 nm in relation to the non-cross-linked control (F/Fo) with different concentrations of Iron(II) in solution applied during cross-linking. As the results in FIG. 5 show, the presence of Iron(II) in riboflavin solution enhances collagen-related fluorescence at 450 nm after exposure to UVA light, indicative of cross-linking activity.

**Cross-linking with Hydrogen Peroxide and Iron(II)**

The following study conducted an investigation to test corneal cross-linking using a 0.1% Iron(II) solution made from $FeSO_4$ solution with a hydrogen peroxide pre-soak. The compositions used in this study do not fall within the scope of the invention.

A. Materials and Method

Pig eyes were shipped overnight on ice from an abattoir (SiouxPreme, Sioux City, IA), rinsed in saline. Eyes were several days old at time of experiment. Eyes were cleaned and epithelium was removed. Corneal flaps (approximately 200 $\mu$m thick) were excised from the eyes with aid of Intralase femtosecond laser (Abbot Medical Optics, Santa Ana, CA). The average thickness of the corneal flaps was calculated as a difference between the measurements before and after the excision from the eyes with an ultrasonic Pachymeter (DGH Technology, Exton, PA).

Corneal flaps were soaked in either distilled water or diluted $H_2O_2$ (1%) for 20 minutes. Flaps soaked in $H_2O_2$ were either rinsed twice with distilled water or removed from $H_2O_2$ and placed in 0.1% $FeSO_4$ solution in distilled water for an additional 20 minute soak followed by a 2X rinse with distilled water. Flaps were dried in a vacuum until the weight change became less than 10% (Rotary vane vacuum pump RV3 A652-01-903, BOC Edwards, West Sussex, UK). Each flap was digested for 2.5 hours at 65°C with 2.5 units/ml of papain (from Papaya latex, Sigma) in 1 ml of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with BBBS and fluorescence of the solutions was measured with excitation of $\lambda$ex = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

Treatments:

| Control: | Corneal flaps were placed in 1.5 mL Eppendorf tubes and soaked with $dH_2O$ for 20 minutes. |
|---|---|
| $H_2O_2$: | Corneal flaps were placed in 1.5 mL Eppendorf tubes and soaked with 1% of $H_2O_2$ for 20 minutes. Corneal flaps were rinsed twice with $dH_2O$ before drying. |
| $H_2O_2$ + Iron(II): | Corneal flaps were placed in 1.5 mL Eppendorf tubes and soaked with 1% of $H_2O_2$ for 20 minutes. Flaps were removed from the original tube and transferred to a new Eppendorf tube with 0.1% Iron(II) solution in $dH_2O$ for 20 minutes. Flaps were rinsed twice with $dH_2O$ before drying. |

B. Results/Conclusion

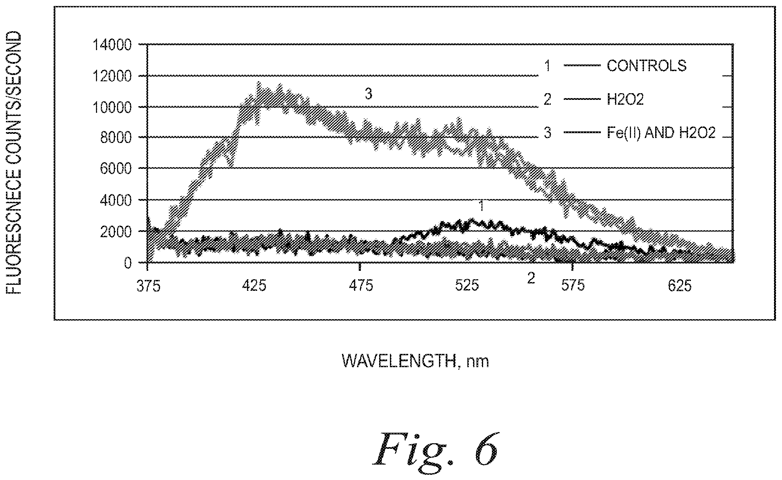

FIG. 6 illustrates fluorescence counts of papain digested corneal flaps treated with: (1) $dH_2O$; (2) $H_2O_2$; and (3) $H_2O_2$ and 0.1% Iron(II). As the results in FIG. 6 show, a fluorescence pattern for the $H_2O_2$ + 0.1% Iron(II) condition is similar to normal cross-linking patterns. This demonstrates that cross-linking occurs when flaps are placed in the Iron solution

after $H_2O_2$, but not when they were exposed to only $H_2O_2$.

**Further Cross-linking with Riboflavin and Iron(II)**

The following study examined the effects of 0.5 mM $FeSO_4$ in 0.1% riboflavin in $dH_2O$ on corneal collagen crosslinking. Samples were either irradiated continuously, or with oxygen and pulsed UVA. The following description combines data from two separate days of experiments. The compositions used in this study do not fall within the scope of the invention.

A. Materials and Methods

Pig eyes were shipped overnight on ice from an abattoir (SiouxPreme, Sioux City, IA), rinsed in saline. Eyes were cleaned and epithelium was removed. Eyes were soaked for 20 minutes with $dH_2O$, 0.1% riboflavin in $dH_2O$ or 0.5 mM $FeSO_4$ in 0.1% riboflavin in $dH_2O$ in an incubator set at 37°C by using a rubber ring to hold the solution on top. If specified, eyes were placed in a beaker with a light oxygen stream for 2 minutes in the incubation chamber prior to irradiation. Corneas were pan-corneally irradiated with a top hat beam (3% root mean square) for the chosen time (4 or 8 minutes) with 365-nm light source (UV LED NCSU033B[T]; Nichia Co., Tokushima, Japan) at the chosen irradiance (30 mW/cm², pulsed or non-pulsed) which was measured with a power sensor (model PD-300-UV; Ophir, Inc., Jerusalem, Israel) at the corneal surface. Corneal flaps (approximately 200 $\mu$m thick) were excised from the eyes with aid of Intralase femtosecond laser (Abbot Medical Optics, Santa Ana, CA). The average thickness of the corneal flaps was calculated as a difference between the measurements before and after the excision from the eyes with an ultrasonic Pachymeter (DGH Technology, Exton, PA). The flaps were placed into a biaxial extensometer (CellScale Biotester 5000, Waterloo, ON), using biorake attachments with 5 tines spanning a width of 3 mm. Each sample was stretched at a constant rate of 4 $\mu$m/s in saline at 37°C until sample failure. The flaps were washed with distilled water 2 times, dried with filter paper, washed with $dH_2O$ two times, and then dried in a vacuum until the weight change became less than 10% (Rotary vane vacuum pump RV3 A652-01-903, BOC Edwards, West Sussex, UK). Each flap was digested for 2.5 hours at 65°C with 2.5 units/ml of papain (from Papaya latex, Sigma) in 1 ml of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with 1XBBBS and fluorescence of the solutions was measured with excitation of $\lambda$ex = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

Treatments:

| | |
|---|---|
| Control: | After being soaked in $dH_2O$, corneal flaps were cut at approximately 200 $\mu$m. |
| 0.1% Riboflavin, CW: | After being soaked in 0.1% riboflavin, eyes were illuminated with 30 mW/cm² of UVA light for 4 minutes, continuous wave (CW). Corneal flaps were cut at approximately 200 $\mu$m. |
| 0.1% Riboflavin + 0.5 mM $FeSO_4$, CW: | After being soaked in 0.1% riboflavin + 0.5mM $FeSO_4$, eyes were illuminated with 30 mW/cm² of UVA light for 4 minutes, continuous wave (CW). Corneal flaps were cut at approximately 200 $\mu$m |
| 0.1% Riboflavin, PW + $O_2$: | After being soaked in 0.1% riboflavin, eyes were placed in a beaker with oxygen stream for 2 minutes, then illuminated with 30 mW/cm² of pulsed UVA light for 8 minutes (1 second on:1 second off) (pulsed wave (PW)). Oxygen was supplied continuously to the beaker during the time of exposure. Corneal flaps were cut at approximately 200 $\mu$m. |
| 0.1% Riboflavin + 0.5 mM $FeSO_4$, PW + $O_2$: | After being soaked in 0.1% riboflavin + 0.5mM $FeSO_4$, eyes were placed in a beaker with oxygen stream for 2 minutes, then illuminated with 30 mW/cm² of pulsed UVA light for 8 minutes (1 second on:1 second off) (pulsed wave (PW)). Oxygen was supplied continuously to the beaker during the time of exposure. Corneal flaps were cut at approximately 200 $\mu$m. |

B. Results

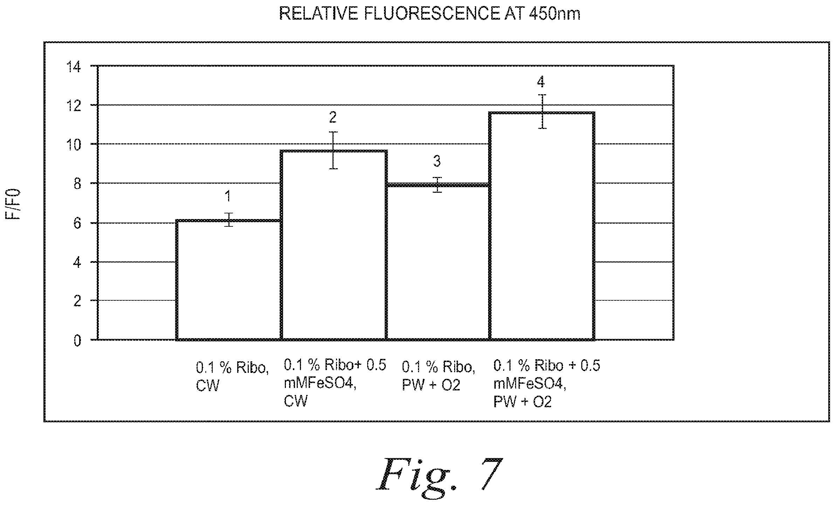

FIG. 7 illustrates fluorescence (relative to untreated controls) recorded at 450 nm for: (1) 0.1% riboflavin (continuous wave (CW)); (2) 0.1% riboflavin + 0.5 mM $FeSO_4$ (CW); (3) 0.1% riboflavin (pulsed wave (PW) + $O_2$); and (4) 0.1% riboflavin + 0.5 mM $FeSO_4$ (PW + $O_2$).

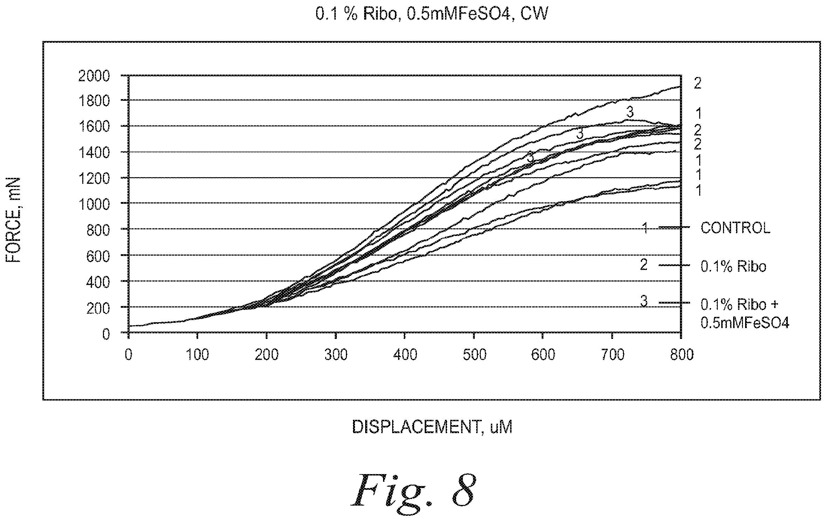

FIG. 8 illustrates average force vs. displacement of each corneal flap measured by tensiometry for: 0.1% riboflavin (CW) (2) and 0.1% riboflavin + 0.5 mM $FeSO_4$ (CW) (3), relative to controls (1).

FIGS. 7-8 illustrate, for repeated experiments, average force vs. displacement of each corneal flap measured by tensiometry for 0.1% riboflavin (PW + $O_2$) (2) and 0.1% riboflavin + 0.5 mM $FeSO_4$ (PW + $O_2$) (3), relative to controls (1).

C. Conclusion

Two methods were used to determine corneal cross-linking in corneal flaps. First, the papain digestion results show an increase in fluorescence under both the continuous (CW) and pulsing (PW) + $O_2$ condition with the addition of $FeSO_4$. The second method, tensiometry, only displayed an increase in biaxial tension for the PW + $O_2$ condition when $FeSO_4$ was added.

The relative fluorescence graph of FIG. 7 shows an increase in fluorescence counts when $FeSO_4$ is added to 0.1% riboflavin as well as when UVA application is changed from a continuous dose to a pulsed dose with oxygen.

FIG. 8 shows the tensiometry results from the continuous UVA dose treatment groups. The 0.1% riboflavin group and the 0.1% riboflavin + 0.5 mM $FeSO_4$ group both display a similar correlation between force and displacement as the displacement increases. Both groups are higher than the control group.

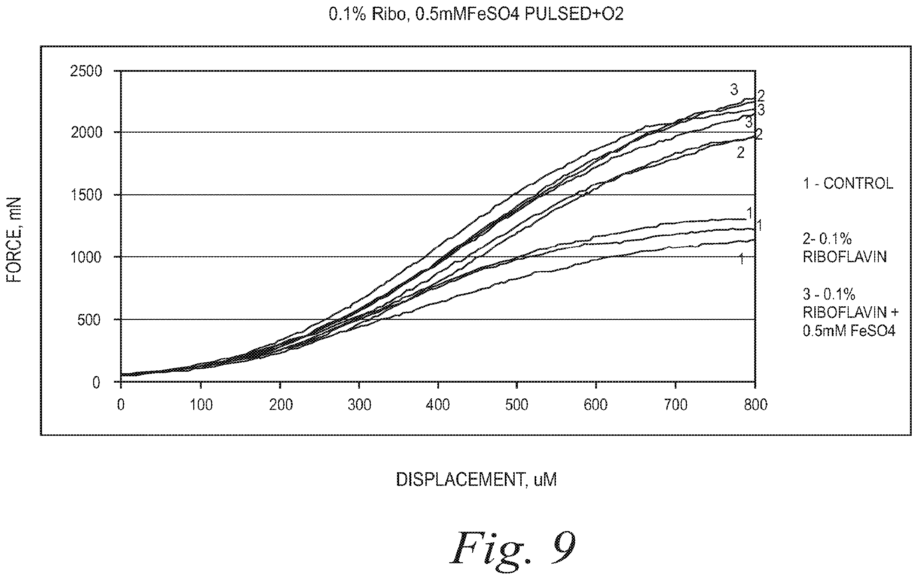

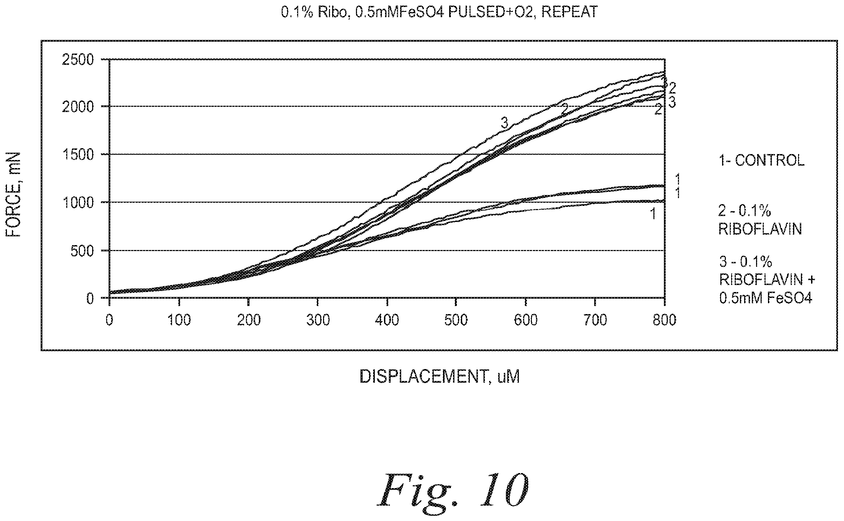

FIGS. 9 and 10 show the tensiometry results from the pulsed UVA application with oxygen treatment groups. The 0.1% riboflavin + 0.5 mM $FeSO_4$ group shows a slightly greater force as the displacement increases. The increase in force was greater for the PW + $O_2$ treatment groups than the increase in the CW treatment groups.

**Cross-linking with Riboflavin with Iron(II) Dissolved in Citrate Buffer**

The following study examined the levels of collagen cross-linking in corneal flaps treated with 0.1% riboflavin in buffer saline solution (available under AVEDRO® PHOTREXA ZD™) versus 0.1% riboflavin in buffer saline solution with Iron(II) dissolved in citrate buffer.

With this treatment, citrate ligands protect ferric and ferrous ions from water and oxygen action which cause low solubility product of ferric hydroxide and other insoluble ferric or ferrous species. While complexation of Iron(II) with citrate might retard the kinetics of Iron(II) oxidation, some iron ions can still participate in Fenton-like reactions in the system studied.

A. Materials and Methods

FeSO4 was added to Citrate Buffer (stock 100mM Citrate Buffer: Citric Acid and 0.33%NaCl in $dH_2O$, adjusted to pH 6.0 with Trisodium Citrate; buffer was diluted to the desired concentration with $dH_2O$). 100 μL of each Fe-Citrate solution was added to 10mL of 0.1% riboflavin in buffer saline solution, for a final pH of 7.1-7.2. Pig eyes were shipped overnight on ice from an abattoir (SiouxPreme, Sioux City, IA), rinsed in saline. Eyes were cleaned and epithelium was removed. Eyes were soaked for 20 minutes with 0.1% riboflavin in buffer saline solution, 0.1% riboflavin in buffer saline solution + 0.25 mM FeSO4 + 0.5mM Citrate Buffer, or 0.1% riboflavin in buffer saline solution + 0.5 mM FeSO4 + 0.25 mM Citrate Buffer in an incubator set at 37°C by using a rubber ring to hold the solution on top. Eyes were placed in a beaker with a light oxygen stream for 2 minutes in the incubation chamber prior to irradiation. Corneas were pan-corneally irradiated with a top hat beam (3% root mean square) for 4 minutes with 365-nm light source (UV LED NCSU033B[T]; Nichia Co., Tokushima, Japan) at the chosen irradiance (30 mW/cm2, pulsed 1 second on: 1 second off) which was measured with a power sensor (model PD-300-UV; Ophir, Inc., Jerusalem, Israel) at the corneal surface. Corneal flaps (approximately 200 μm thick) were excised from the eyes with aid of Intralase femtosecond laser (Abbot Medical Optics, Santa Ana, CA). The average thickness of the corneal flaps was calculated as a difference between the measurements before and after the excision from the eyes with an ultrasonic Pachymeter (DGH Technology, Exton, PA). The flaps were washed with distilled water 2 times, dried with filter paper, washed with $dH_2O$ 2 times, and then dried in a vacuum until the weight change became less than 10% (Rotary vane vacuum pump RV3 A652-01-903, BOC Edwards, West Sussex, UK). Each flap was digested for 2.5 h at 65°C with 2.5 units/ml of papain (from Papaya latex, Sigma) in 1 ml of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with 1XBBBS and fluorescence of the solutions was measured with excitation of $\lambda ex$ = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

Treatments:

Control: After being soaked in 0.1% riboflavin in buffer saline solution for 20 minutes, corneal flaps were cut at approximately 200 $\mu$m.

0.1% riboflavin (buffer saline solution), PW + $O_2$:
After being soaked in 0.1% riboflavin in buffer saline solution for 20 minutes, eyes were placed in a beaker with a light oxygen stream for 2 minutes, then illuminated with 30 mW/cm2 of pulsed UVA light for 4 minutes (1 second on:1 second off) with oxygen. Corneal flaps were cut at approximately 200 $\mu$m.

0.1% riboflavin (buffer saline solution) + 0.25mM Iron + 0.5mM Citrate Buffer PW + $O_2$:
After being soaked in 0.1% riboflavin in buffer saline solution + 0.25mM Iron + 0.5mM Citrate Buffer for 20 minutes, eyes were placed in a beaker with a light oxygen stream for 2 minutes, then illuminated with 30 mW/cm2 of pulsed UVA light for 4 minutes (1 second on:1 second off) with oxygen. Corneal flaps were cut at approximately 200 $\mu$m.

0.1% riboflavin (buffer saline solution) + 0.5mM Iron+ 0.25mM Citrate Buffer PW + $O_2$:
After being soaked in 0.1% riboflavin in buffer saline solution + 0.5mM Iron + 0.25mM Citrate Buffer for 20 minutes, eyes were placed in a beaker with a light oxygen stream for 2 minutes, then illuminated with 30 mW/cm2 of pulsed UVA light for 4 minutes (1 second on:1 second off) with oxygen. Corneal flaps were cut at approximately 200 $\mu$m.

B. Results

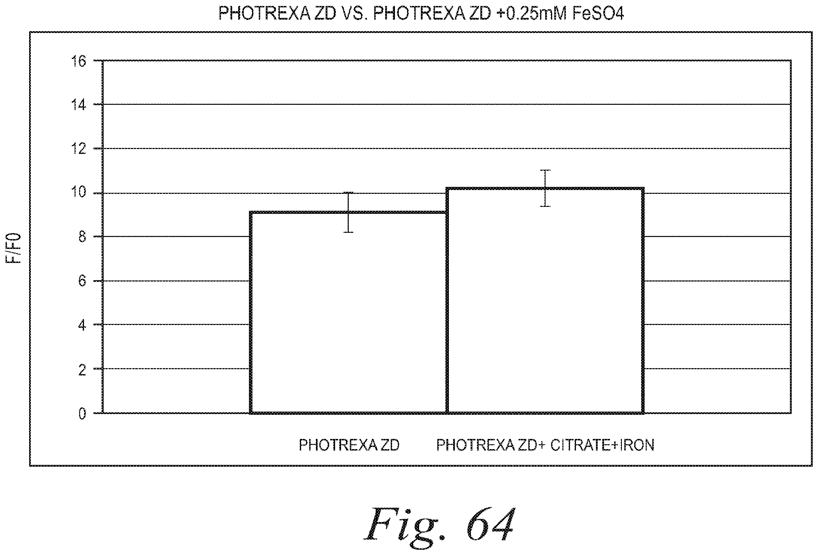

FIG. 64 illustrates the relative fluorescence of the cross-linked flaps with 0.1% riboflavin (buffer saline solution) versus 0.1% riboflavin (buffer saline solution) + 0.25 mM Iron + 0.5mM Citrate Buffer, where the eyes were illuminated with 30 mW/cm2 of CW UVA light for 4 minutes and pulsed with oxygen.

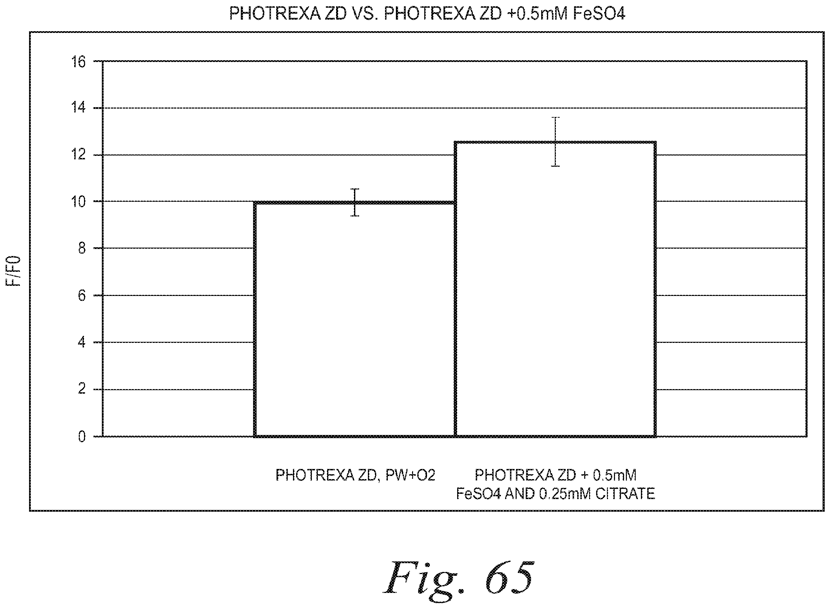

FIG. 65 illustrates the relative fluorescence of the cross-linked flaps with 0.1% riboflavin (buffer saline solution) versus 0.1% riboflavin (buffer saline solution) + 0.5mM Iron + 0.25mM Citrate Buffer, where the eyes were illuminated with 30 mW/cm2 of CW UVA light for 4 minutes, pulsed with oxygen.

C. Conclusion

Iron (II) Sulfate was dissolved in Citrate Buffer at either 25mM FeSO4 in 50mM Citrate Buffer or 50mM FeSO4 in 25mM Citrate Buffer. For both solutions, 100uL was added to 10mL of 0.1% riboflavin (buffer saline solution) to reach the desired concentration. In both cases, there was no visible precipitation, and the solution remained stable at room temperature for an extended period of time.

0.1% riboflavin (buffer saline solution) with 0.25mM FeSO4 had a slightly higher average fluorescence counts from Papain digestion than 0.1% riboflavin (buffer saline solution) alone. 0.1% riboflavin (buffer saline solution) with 0.5mM FeSO4 had about 26% higher fluorescence counts from Papain digestion than 0.1% riboflavin (buffer saline solution) alone.

**Cross-linking with Riboflavin and 2,3-Butanedione**

Diacetyl (2,3-butanedione) is an $\alpha$-diketone that is present naturally in butter and a variety of foods including dairy products and alcoholic beverages as a product of bacterial fermentation. The U.S. Food and Drug Administration granted diacetyl GRAS (generally recognized as safe) status as a direct food ingredient, and consumption of the low levels of diacetyl present in food has not been reported to present a human health risk.

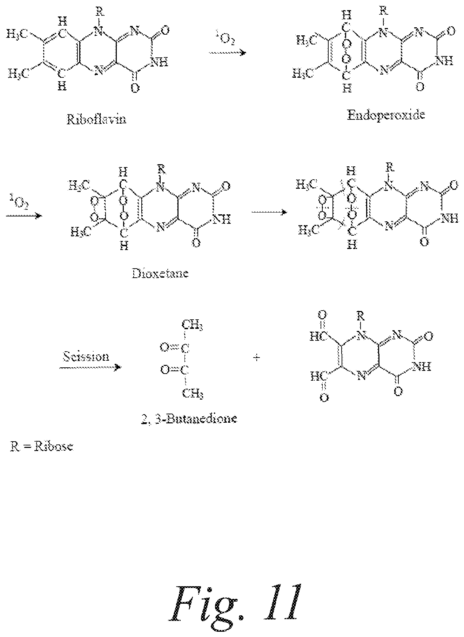

According to studies, 2,3-butanedione is a major volatile product detected in the riboflavin solutions after irradiation with UV light. The mechanism includes the interaction between singlet oxygen and riboflavin. FIG. 11 illustrates the mechanism for the formation of 2,3-butanedione from riboflavin and singlet oxygen. Studies confirm generation of 2,3-butanedione in riboflavin solution after UV irradiation and its participation in corneal cross-linking has been proposed. The following study conducted an investigation to measure quantitatively the cross-linking efficiency of 2,3-butanedione when using it for corneal cross-linking with and without riboflavin. The compositions used in this study do not fall within the scope of the invention.

A. Materials and Methods

Pig eyes were shipped overnight on ice from an abattoir (SiouxPreme, Sioux City, IA), rinsed in saline. Eyes were cleaned and epithelium was removed. Eyes were soaked for 20 minutes with 0.1% riboflavin in $dH_2O$, or 0.1% 2,3-butanedione (BD) in $dH_2O$ in an incubator set at 37°C by using a rubber ring to hold the solution on top of the eye. Corneas were pan-corneally irradiated with a top hat beam (3% root mean square) for 4 minutes with 365-nm light source (UV LED NCSU033B[T]; Nichia Co., Tokushima, Japan) at irradiance 30 mW/cm$^2$, which was measured with a power sensor (model PD-300-UV; Ophir, Inc., Jerusalem, Israel) at the corneal surface. Corneal flaps (approximately 200 $\mu$m thick) were excised from the eyes with aid of Intralase femtosecond laser (Abbott Medical Optics, Santa Ana, CA). The average thickness of the corneal flaps was calculated as a difference between the measurements before and after the excision from the eyes with an ultrasonic Pachymeter (DGH Technology, Exton, PA). The flaps were then placed into a biaxial extensometer (CellScale Biotester 5000, Waterloo, ON), using biorake attachments with 5 tines spanning a width of 3 mm. Each sample was stretched at a constant rate of 4 $\mu$m/s in saline at 37°C until sample failure. The flaps were washed with distilled water, dried in a vacuum until the weight change became less than 10% (Rotary vane vacuum pump RV3 A652-01-903, BOC Edwards, West Sussex, UK). Each flap was digested for 2.5 h at 65°C with 2.5 units/ml of papain (from Papaya latex, Sigma) in 1 ml of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with 1XBBBS and fluorescence of the solutions was measured with excitation of $\lambda$ex = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

B. Results/Conclusion

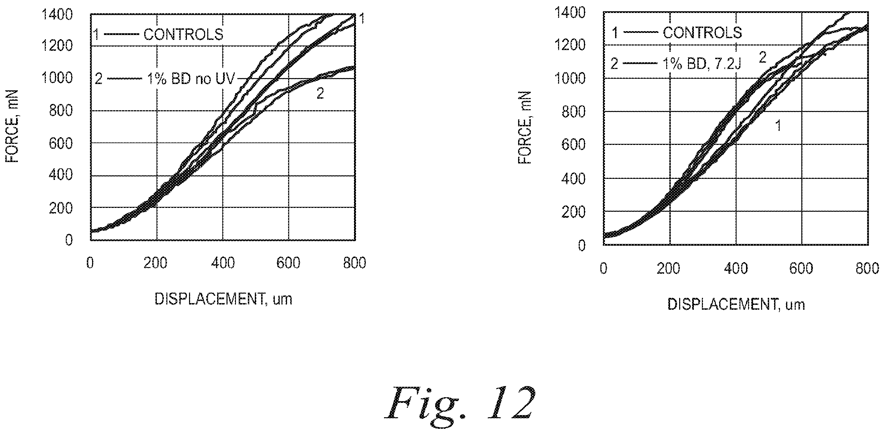

FIG. 12 illustrates displacement-force diagrams for corneal flaps treated with 1% solution of 2,3-butanedione (BD) in $dH_2O$ without ultraviolet (UV) light (left panel) and with UV light (right panel) (365 nm, 30 mW for 4 min).

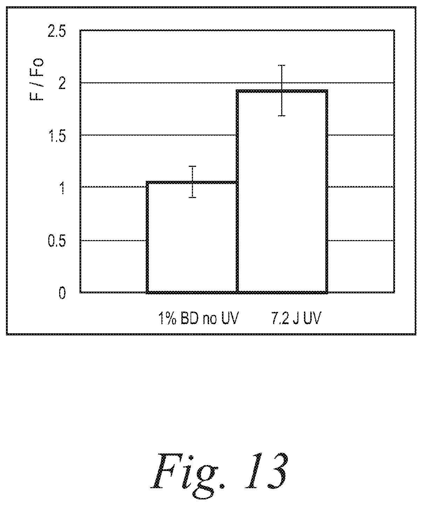

FIG. 13 illustrates fluorescence (relative to the untreated controls, Fo) recorded at 450 nm from the papain digested 200 $\mu$m-thick corneal flaps, treated with 1% solution of BD with and without UV light.

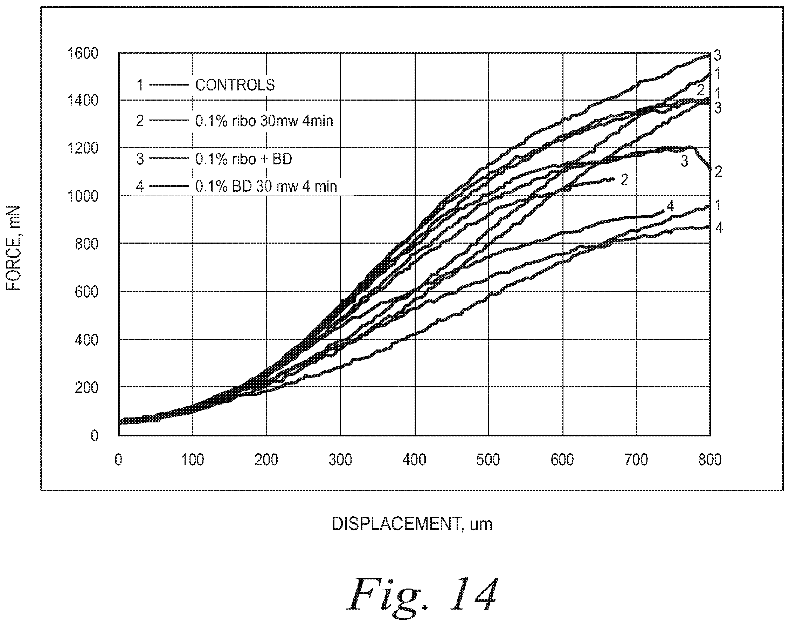

FIG. 14 illustrates displacement-force diagrams for corneal flaps treated with UV light (365 nm, 30 mW for 4 min) and: 0.1% solution of riboflavin in $dH_2O$ (1); 0.1% BD in $dH_2O$ (4); and mixture of 0.1% riboflavin and 0.1% BD in $dH_2O$ (3), relative to controls (1).

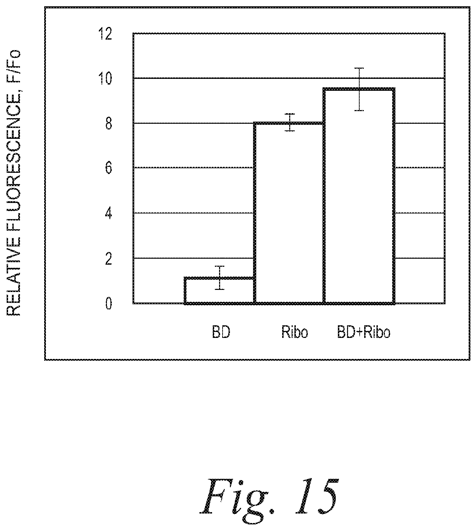

FIG. 15 illustrates fluorescence (relative to the untreated controls, Fo) recorded at 450 nm from the papain digested 200 $\mu$m-thick corneal flaps, treated with 30 mW UVA for 4 min and: 0.1% solution of riboflavin in $dH_2O$; 0.1% BD in $dH_2O$; and mixture of 0.1% riboflavin and 0.1% BD in $dH_2O$.

As shown in FIGS. 12 and 13, 2,3-butanedione itself (without UV light) does not cross-link corneal flaps, but when irradiated with 365 nm UV light, it leads to the increase of the corneal stiffness and fluorescence output recorded from the treated cornea. FIGS. 14 and 15 show change in the stiffness of the corneal flaps when mixture of BD with riboflavin is used for the cross-linking.

Accordingly, 2,3-butanedione can be used as an additive to a riboflavin formulation to increase cross-linking efficacy.

Based on its participation in corneal cross-linking described above, it is also contemplated that 2,3-butanedione can also be used as a primary cross-linking agent (without riboflavin).

**Cross-linking with Products from Hydrolysis of Riboflavin**

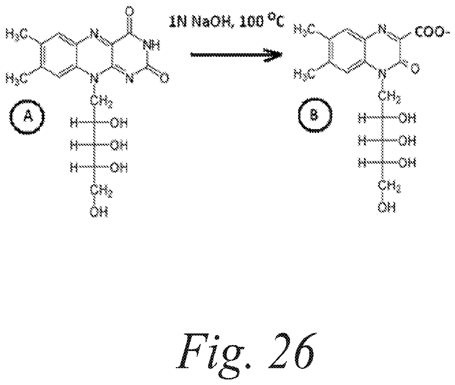

Riboflavin is hydrolyzed in alkaline solution to give urea and 1,2-dihydro-6,7-dimethyl-2-keto-1-D-ribityl-3-quinoxaline-carboxylic acid among the hydrolysis products. FIG. 26 illustrates alkaline hydrolysis of riboflavin (A) into 1,2-dihydro-6,7-dimethyl-2-keto-1-D-ribityl-3-quinoxaline-carboxylic acid (B).

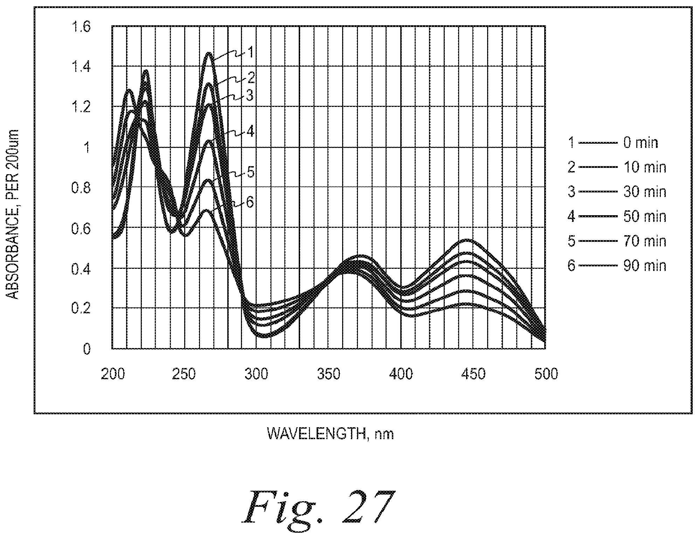

The kinetics of the alkaline degradation has been followed spectrophotometrically and it has been noted that the optical density of the original riboflavin solution decreases at 450 and 370 nm but increases at 310 nm. When the present inventors heated 0.1% riboflavin-5-phosphate solution in 0.85% blood buffered bank saline (BBBS) (Thermo Scientific) at 120 $^0$C, they detected a similar development (as shown in FIG. 27). In particular, FIG 27 illustrates UV/Visible (Vis) light spectra of the 0.1% riboflavin-5-phosphate in BBBS kept at 120°C for different amounts of time.

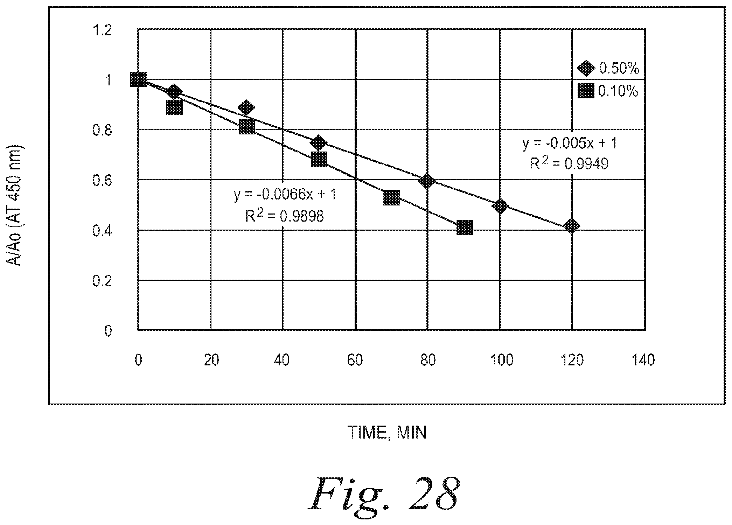

During the heating procedure, concentration of riboflavin decreases with time (FIG. 27, see absorbance change at 450 nm). The rate of destruction of riboflavin depends also on its concentration in solution. For example, the present inventors have found that a 0.1% solution hydrolyzes 1.3 times more quickly than a 0.5% solution (as shown in FIG 28). In particular, FIG. 28 illustrates the rate of hydrolysis of riboflavin at 120°C in BBBS as measured by absorbance at 450 nm (0.1% solution and 0.5% solution, $A_0$ is the absorbance before heating).

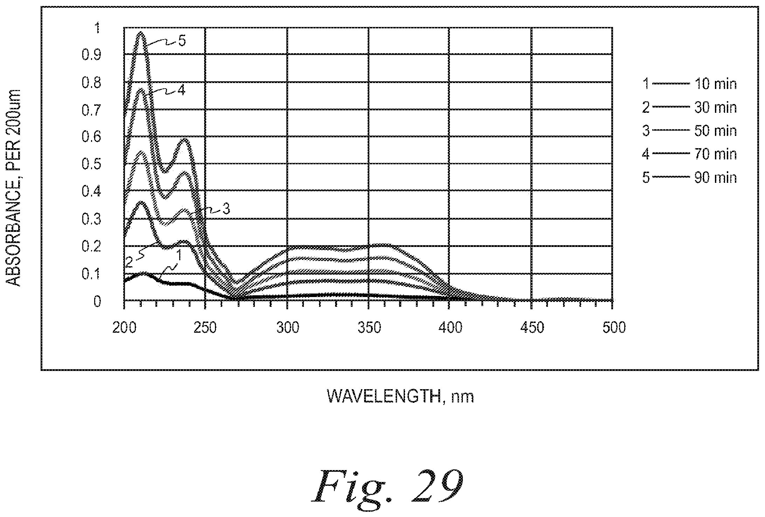

At the same time, there is an accumulation of products resulting from the hydrolysis (as shown in FIG. 29). In particular, FIG. 29 illustrates UV/Vis spectra which is obtained from FIG. 27 by subtracting absorbance of the remaining riboflavin.

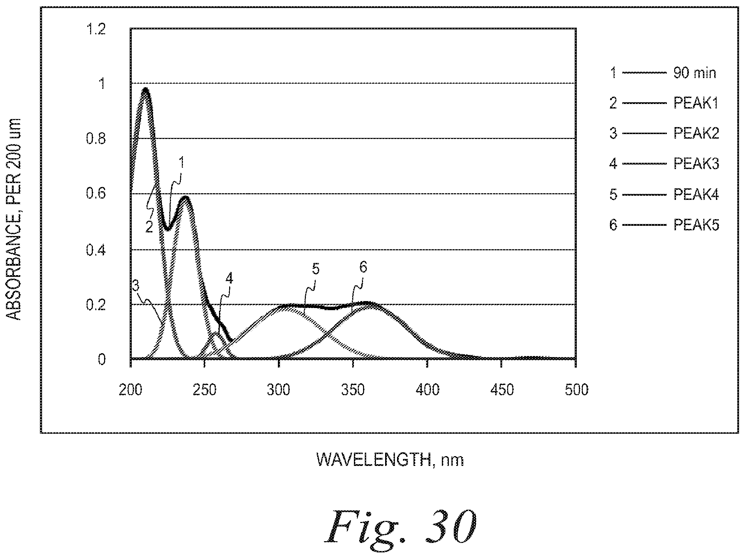

It is possible to analyze spectra from FIG. 29 by combining Gaussian absorption peak shapes (as shown in FIG. 30). In particular, FIG. 30 illustrates spectral analysis of the hydrolyzed solution after 90 min at 120°C (absorbance of residual riboflavin was subtracted from the analyzed spectrum).

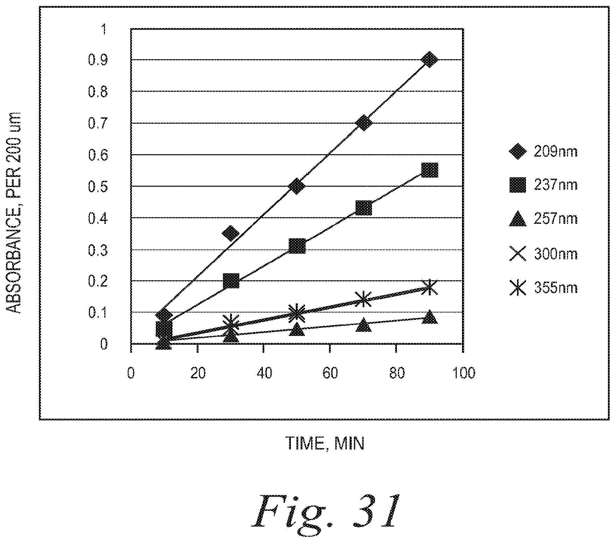

Accumulation of the products during the riboflavin hydrolysis follows by the linear increase in absorption at 209, 237, 257, 300, and 355 nm (as shown in FIG. 31). In particular, FIG. 31 illustrates change in the absorbance of the different peaks during the time of hydrolysis.

For HPLC (high-pressure liquid chromatography) analysis of the degradation products of riboflavin, a Dionex UltiMate 3000 with a Lichrospher WP300 RP18 column, 250 mm × 4.0 mm, 5 $\mu$m from Merck Millipore was used. Mobile phase (A) was water containing monobasic potassium phosphate (7.35 g/L) and (B) methanol. In general, isocratic conditions (15% B, flow of 1.70 mL/min, 30 min, 40 °C) were suitable for analysis of the degradation process. UV spectra were obtained during the HPLC analysis using a diode array detector ($\lambda$ = 200-450nm) and the chromeleon software.

A procedure with water (A) as mobile phase and acetonitrile (B) with various TFA concentrations was successfully used for final product analysis (vide supra) but failed for the analysis of the degradation process.

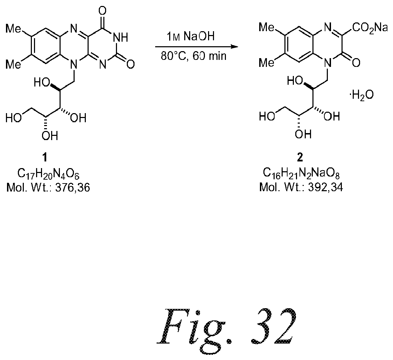

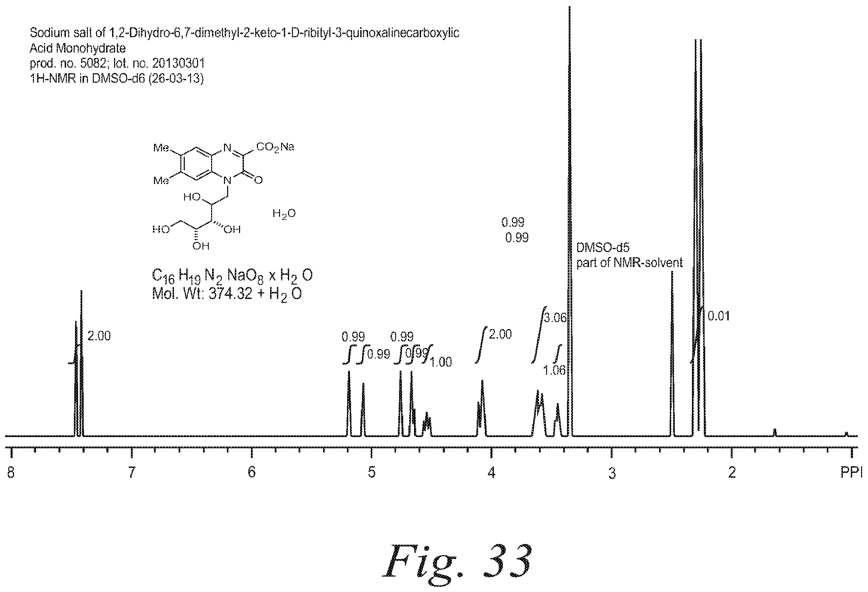

For cross-linking treatments, the sodium salt of 1,2-Dihydro-6,7-dimethyl-2-keto-1-D-ribityl-3-quinoxalinecarboxylic acid monohydrate 2, an early stage alkaline degradation product of riboflavin, as shown in FIG. 32, can be synthesized. The quinoxaline 2 can be prepared by using a synthetic protocol from Surrey et al. J. Am. Chem. Soc. 1951, 73, 3236-2338. FIG. 33 illustrates NMR spectrum of the synthesized riboflavin degradation product 2.

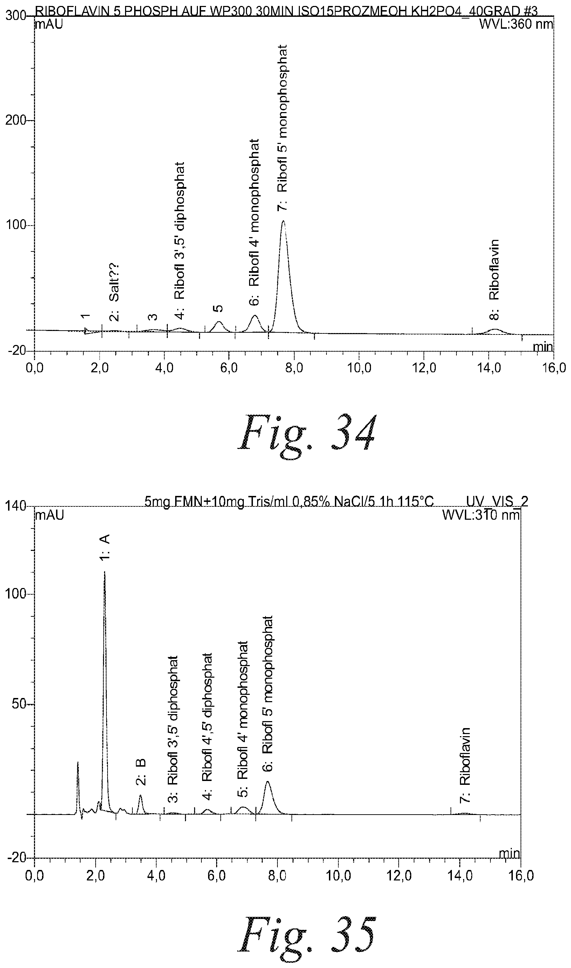

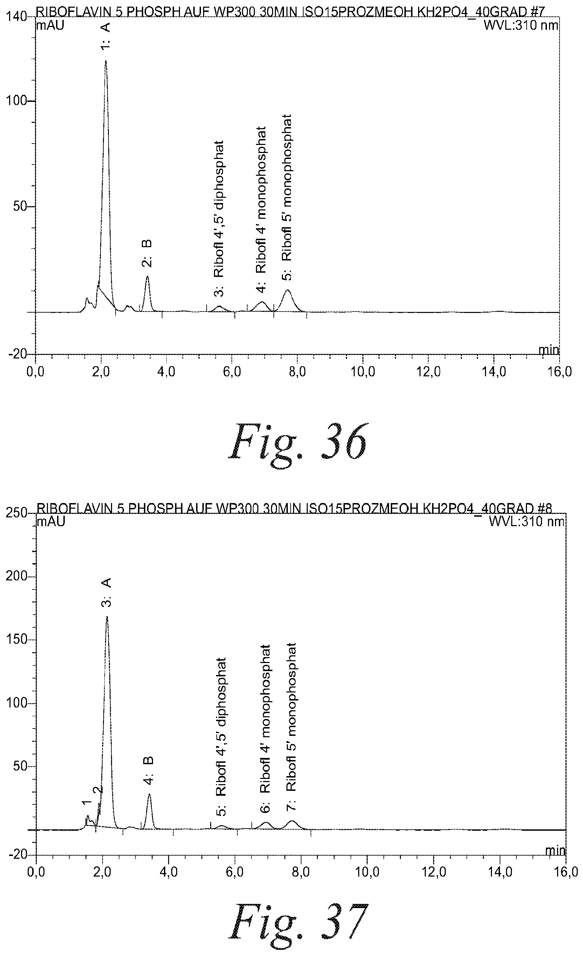

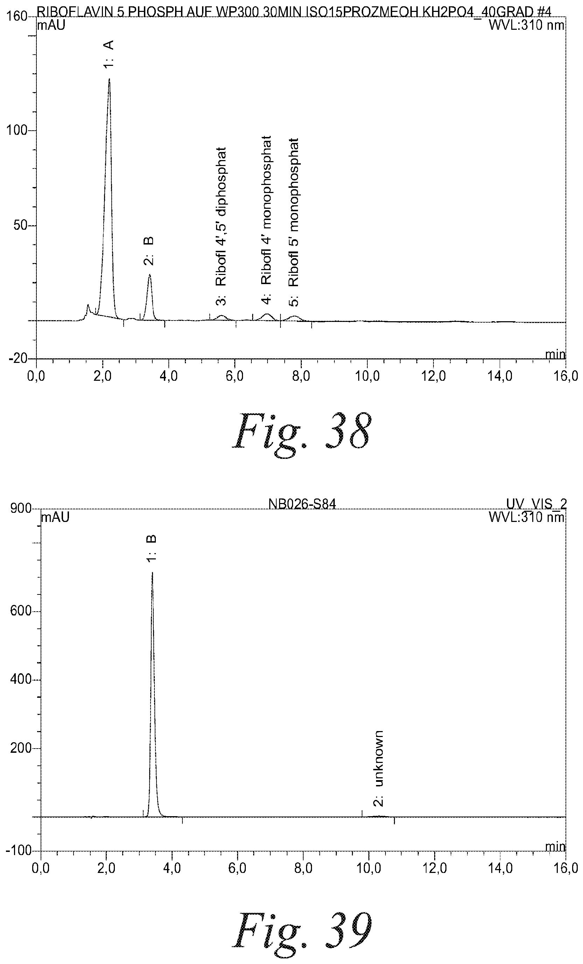

Degradation experiments proved that the synthesized compound 2 is also produced by thermal degradation of a monophosphorylated riboflavin (5-FMN) and corresponds to peak B in FIGS. 35 to 39. FIG. 34 illustrates monophosphorylated riboflavin (5-FMN) in buffered blood bank saline without thermal treatment. FIG. 35 illustrates 5-FMN in buffered blood bank saline after 1 hour of thermal treatment. FIG. 36 illustrates 5-FMN in buffered blood bank saline after 2 hours of thermal treatment. FIG. 37 illustrates 5-FMN in buffered blood bank saline after 3 hours of thermal treatment. FIG. 38 illustrates 5-FMN in buffered blood bank saline after 4 hours of thermal treatment. FIG. 39 illustrates HPLC trace of the synthesized riboflavin degradation product 2.

A second degradation product A is also produced during the thermal degradation of 5-FMN. Due to its more polar characteristics (shorter retention time) it is assumed that this peak corresponds to the phosphorylated quinoxaline compound. This assumption can be confirmed by comparing the UV spectra of both compounds. The similarity of the spectra indicates that no change at the chromophore has taken place. Therefore, it is assumed that this quinoxaline intermediate is formed by the loss of one molecule of urea without the hydrolysis of the phosphorous ester.

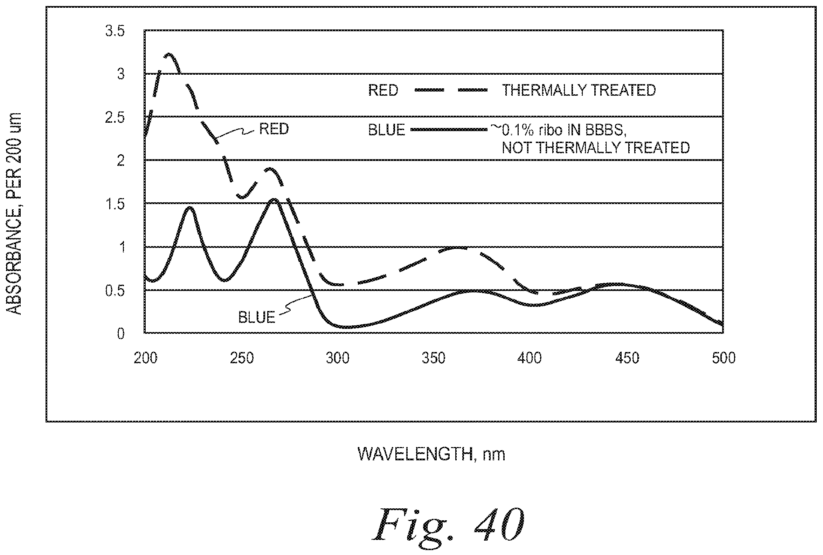

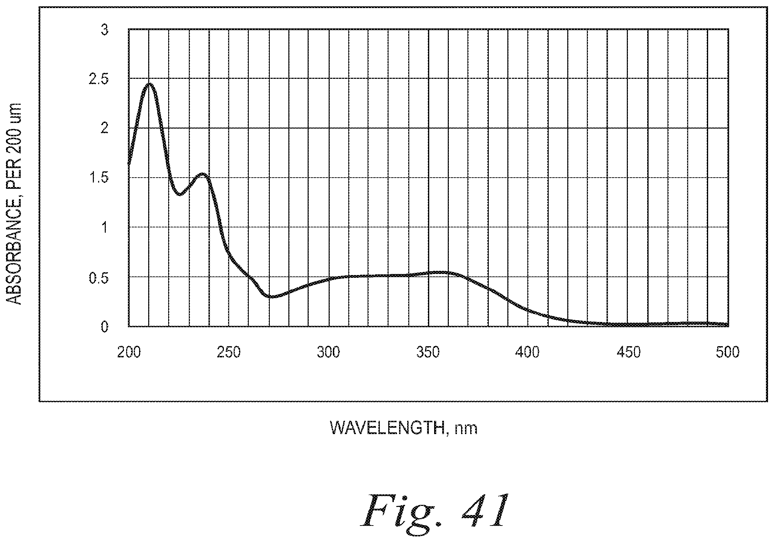

Riboflavin-5-phosphate solution (0.5% riboflavin) in 0.85% Blood Bank Buffered Saline (Thermo Scientific) was sealed in a plastic container and kept for 2 hours at 120 $^0$C. Absorption of this solution was measured after the heat treatment and compared to the absorption of not treated solution (containing ~0.1% riboflavin) in 0.85% BBBS (pH of the solutions = 6.6 for thermally treated and 6.9 for not treated). FIG. 40 illustrates absorption spectra of thermally heated (red line) and not heated (blue line) riboflavin solutions (recorded in a quartz cuvette with 200 $\mu$m optical path). FIG. 41 illustrates the Difference between two spectra in FIG. 34.

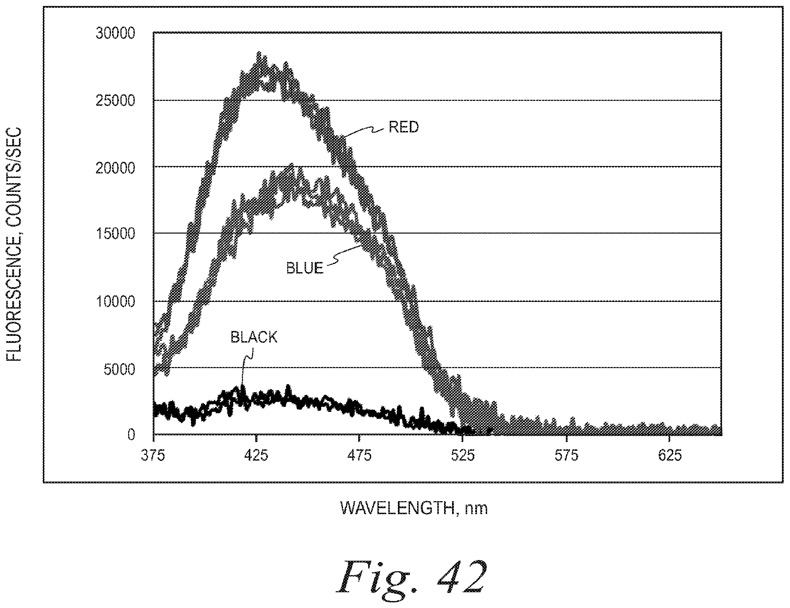

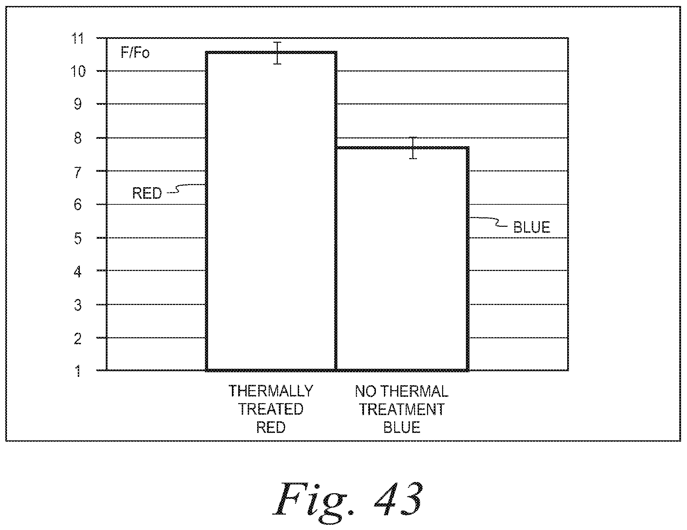

The compositions of the following study do not fall within the scope of the invention. two riboflavin solutions were used for cross-linking of porcine corneas (no epithelium, 20 min soak, 30 mW/cm² for 4 min continuous UVA exposure without application of riboflavin drops during the irradiation). Flaps were cut with the average thickness of 200 $\mu$m. Fluorescence of the digested with papain buffer corneas are presented on FIG. 42. In particular, FIG. 42 illustrates fluorescence of the digested corneas: non-cross-linked control (black line), cross-linked with 0.1% riboflavin which was not heated (blue line), cross-linked with thermally treated riboflavin solution (red line). FIG. 43 illustrates relative fluorescence of the cross-linked corneal samples: red - using thermally treated riboflavin solution, blue - using not heated 0.1% riboflavin solution.

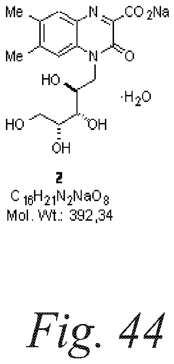

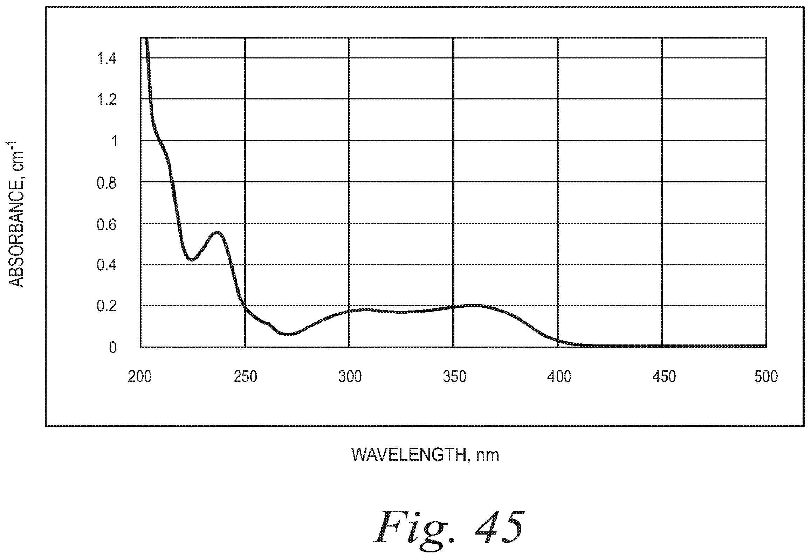

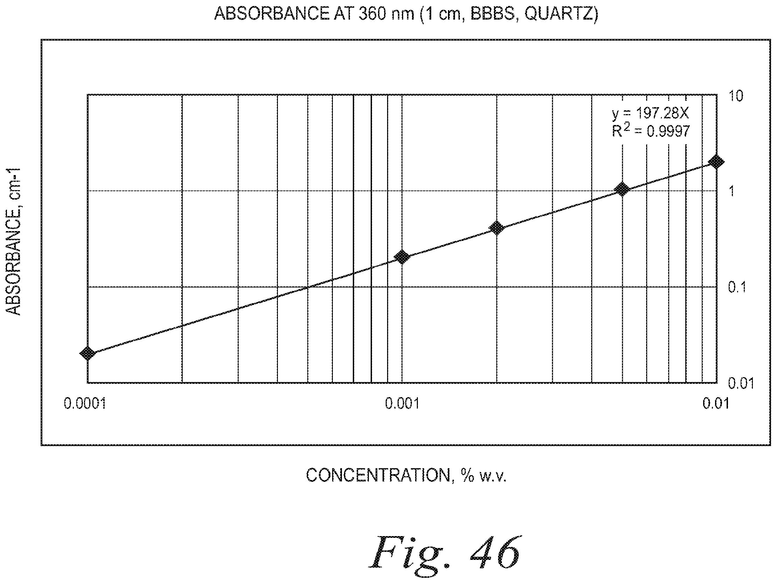

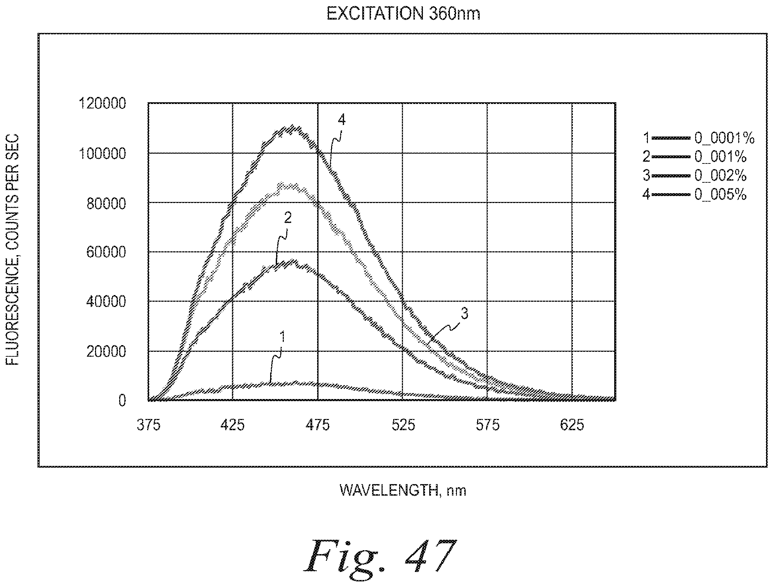

Sodium salt of 1,2-Dihydro-6,7-dimethyl-2-keto-1-D-ribityl-3-quinoxalinecarboxylic acid monohydrate, as shown in FIG. 44, has a strong absorbance at 360 nm (as shown in FIGS. 45 and 46) and noticeable fluorescence with a maximum around 460 nm (as shown in FIG. 47). FIG. 45 illustrates absorbance spectrum of 0.001% solution of sodium salt of 1,2-Dihydro-6,7-dimethyl-2-keto-1-D-ribityl-3-quinoxalinecarboxylic acid monohydrate in BBBS (quartz, 1 cm light path) FIG. 46 illustrates UV absorbance of sodium salt of 1,2-Dihydro-6,7-dimethyl-2-keto-1-D-ribityl-3-quinoxalinecarboxylic acid monohydrate at 360 nm (solution in BBBS, quartz cuvette with 1 cm light path). FIG. 47 illustrates fluorescence of sodium salt of 1,2-Dihydro-6,7-dimethyl-2-keto-1-D-ribityl-3-quinoxalinecarboxylic acid monohydrate in BBBS solutions (excitation 360 nm).

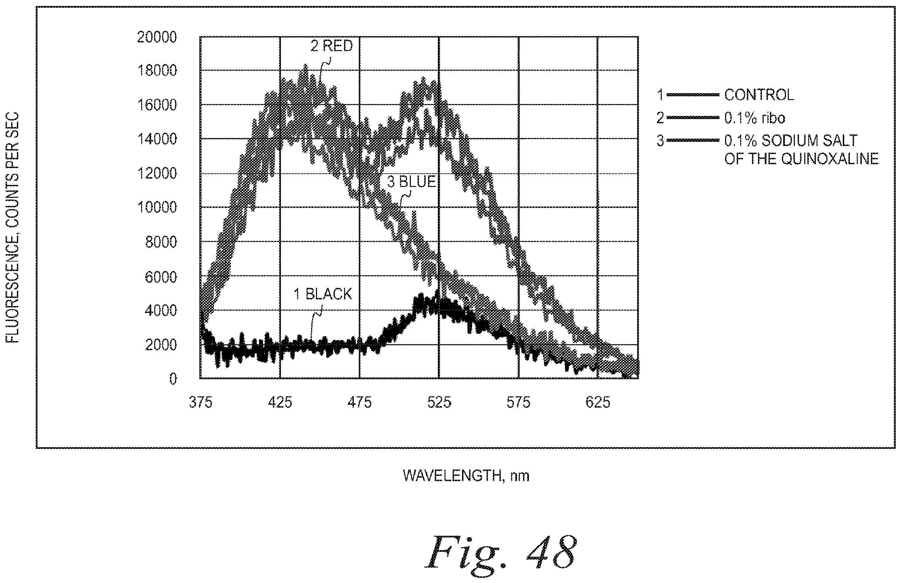

The compositions of the following study do not fall within the scope of the invention. Solutions of 0.1% riboflavin-5-phosphate and 0.1% sodium salt of 1,2-Dihydro-6,7-dimethyl-2-keto-1-D-ribityl-3-quinoxalinecarboxylic acid monohydrate in BBBS were used for cross-linking of the porcine corneas (no epithelium, 20 min soak, 30 mW/cm² for 4 min continuous UVA exposure without application of riboflavin or other cross-linker drops during the irradiation). Flaps were cut with the average thickness of 200 $\mu$m. Fluorescence of the digested with papain buffer corneas are presented. Corneas cross-linked with riboflavin or sodium salt of 1,2-Dihydro-6,7-dimethyl-2-keto-1-D-ribityl-3-quinoxalinecarboxylic acid monohydrate both demonstrated elevated fluorescence in the area 450 nm (as shown in FIG. 48). FIG. 48 illustrates fluorescence of the digested corneas: non-cross-linked control (black line), cross-linked with 0.1% riboflavin in BBBS (red line), cross-linked with 0.1% sodium salt of 1,2-Dihydro-6,7-dimethyl-2-keto-1-D-ribityl-3-quinoxalinecarboxylic acid monohydrate (blue line).

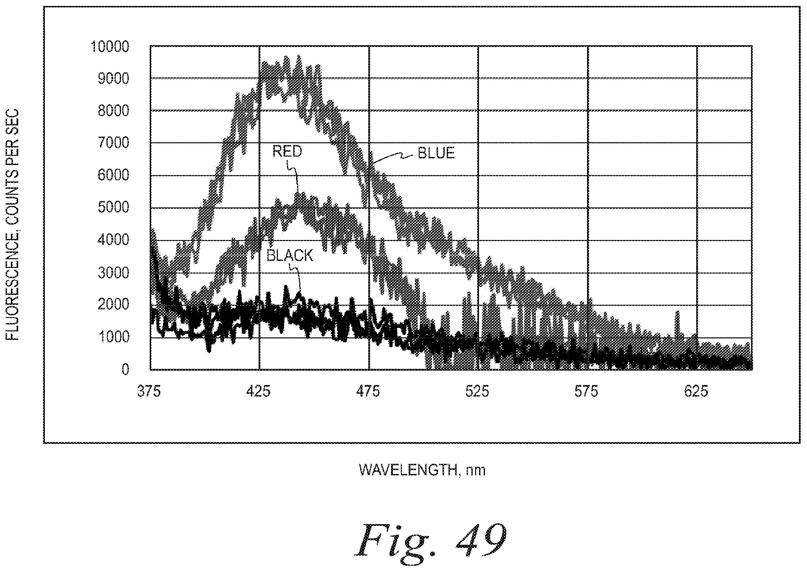

The compositions of the following experiments do not fall within the scope of the invention. 200 $\mu$m-thick corneal flaps without epithelium were cut off from the porcine eyes, placed in 1 mL 0.1% solution of riboflavin in BBBS or 0.1% solution of sodium salt of 1,2-Dihydro-6,7-dimethyl-2-keto-1-D-ribityl-3-quinoxalinecarboxylic acid monohydrate in BBBS, de-oxygenated by bubbling argon for 3 min, then sealed between quartz sheets and irradiated for 4 min at 30 mW/cm$^2$ in $CO_2$ atmosphere. Flaps were rinsed in distilled water, vacuum-dried and digested in the papain buffer. Fluorescence of the obtained solutions was recorded at excitation of 360 nm in order to evaluate collagen fluorescence (as shown in FIG. 49). FIG. 49 illustrates fluorescence of the digested corneal flaps: non-cross-linked control (black line), cross-linked with 0.1% riboflavin in BBBS (red line), cross-linked with 0.1% sodium salt of 1,2-Dihydro-6,7-dimethyl-2-keto-1-D-ribityl-3-quinoxalinecarboxylic acid monohydrate (blue line).

Fluorescence of the corneal flaps which were cross-linked with sodium salt of 1,2-Dihydro-6,7-dimethyl-2-keto-1-D-ribityl-3-quinoxalinecarboxylic acid monohydrate was higher than the fluorescence of the corneal flaps cross-linked with riboflavin. This suggests a lower sensitivity to the oxygen presence when sodium salt of 1,2-Dihydro-6,7-dimethyl-2-keto-1-D-ribityl-3-quinoxalinecarboxylic acid monohydrate is used as a cross-linking agent. Thus, according to aspects of the present disclosure, this salt may be employed in treatments where lower sensitivity to the presence of oxygen is advantageous.

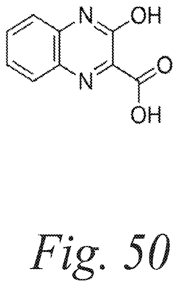

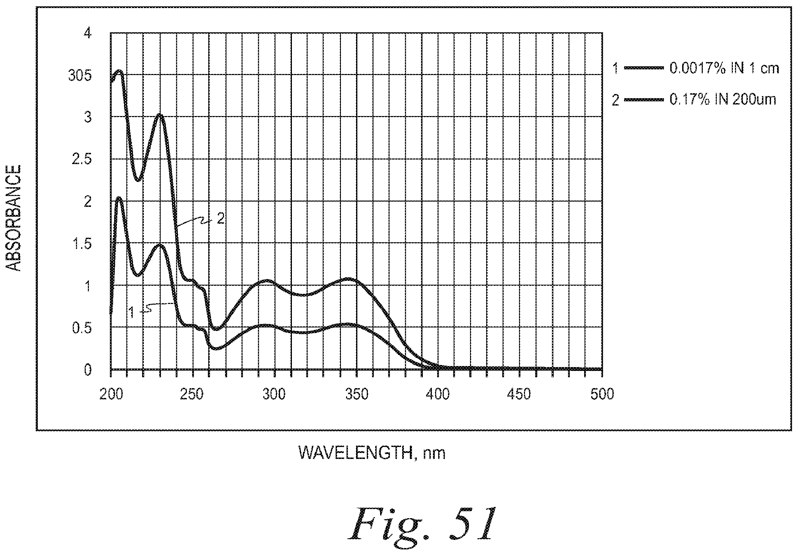

0.17% and 0.017% solutions of 3-hydroxy-2-quinoxalinecarboxylic acid, as shown in FIG. 50, were prepared in BBBS and recorded absorbance (in 200 $\mu$m and 1 cm - thick quartz quvettes) as presented in the FIG. 51. FIG. 51 illustrates absorbance spectra of 3-hydroxy-2-quinoxalinecarboxylic acid.

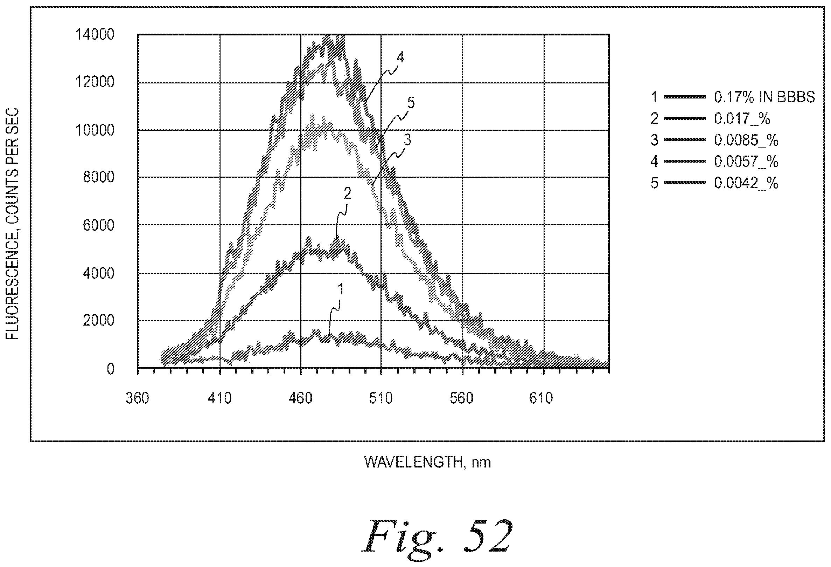

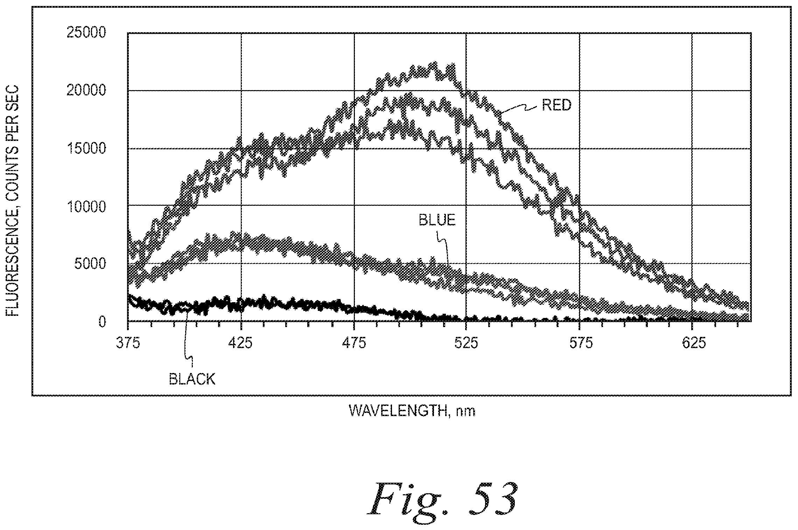

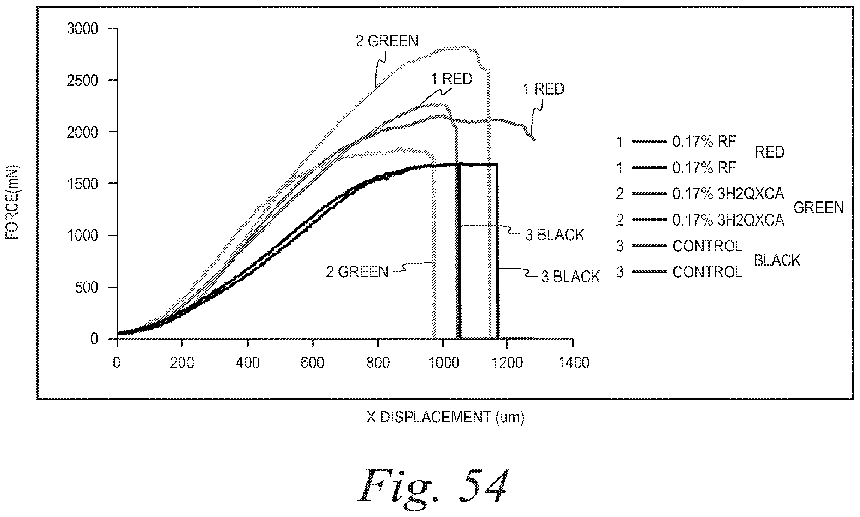

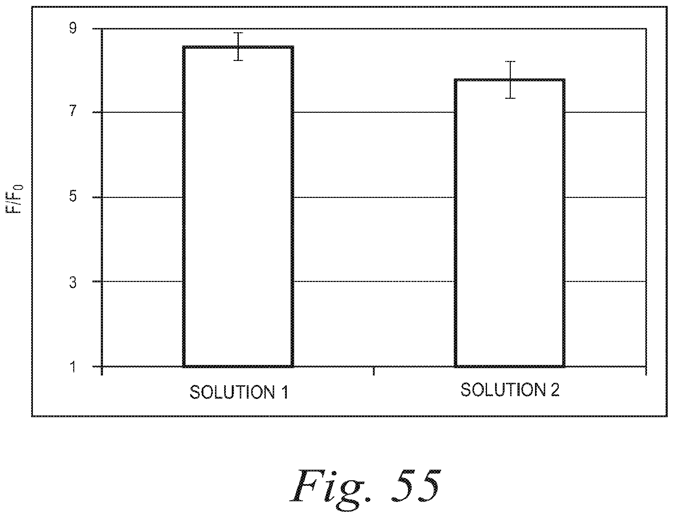

0.17% solution of 3-hydroxy-2-quinoxalinecarboxylic acid exhibited a strong fluorescence quenching (as shown in FIG. 52) because upon dilution, its fluorescence increased significantly. FIG. 52 illustrates fluorescence of 3-hydroxy-2-quinoxalinecarboxylic acid's solutions with different concentrations in BBBS, recorded with excitation of 360 nm. FIG. 53 illustrates fluorescence of the papain digested corneal flaps (200 $\mu$m thick) cross-linked with 0.17% (red lines) and 0.017% (blue lines) solutions of 3-hydroxy-2-quinoxalinecarboxylic acid in BBBS (no epithelium, 20 min soaking time, 30 mW/cm$^2$ for 4 min), relative to non-cross-linked controls (black lines). FIG. 54 illustrates tensiometry plots of 200- $\mu$m thick corneal flaps irradiated at 30 mW/cm2 for 4 min, preliminary saturated for 20 min with 0.17% riboflavin (red lines) and 0.17% 3-hydroxy-2-quinoxalinecarboxylic acid (3H2QXCA, green lines)relative to non-cross-linked controls (black lines). FIG. 55 illustrates relative fluorescence of the papain digested corneal flaps (200 $\mu$m thick) cross-linked with 0.1% riboflavin (blue bar, solution 2) and 0.1% riboflavin containing 0.02% solution of 3-hydroxy-2-quinoxalinecarboxylic acid in BBBS (red bar, solution 1) (no epithelium, 20 min soaking time, 30 mW/cm$^2$ for 4 min), where $F_0$ - fluorescence of a non-cross-linked flap. The compositions discussed in this paragraph do not fall within the scope of the invention.

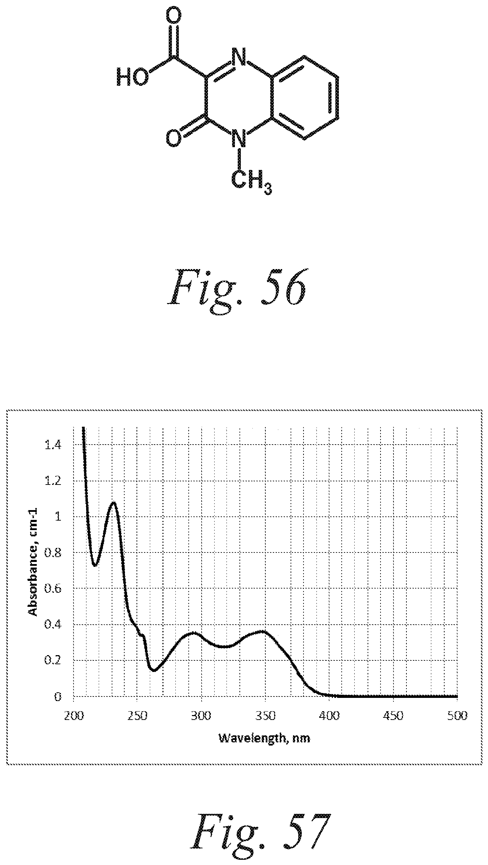

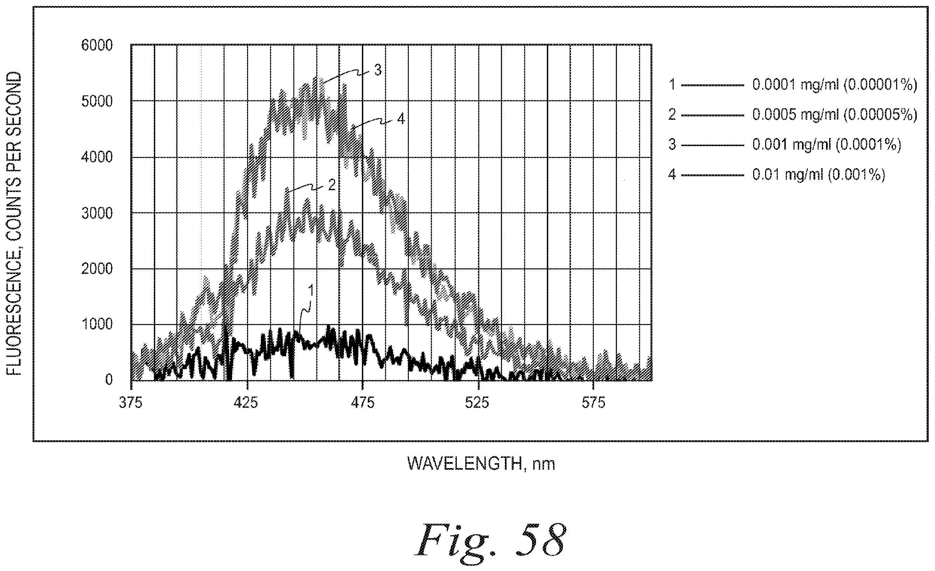

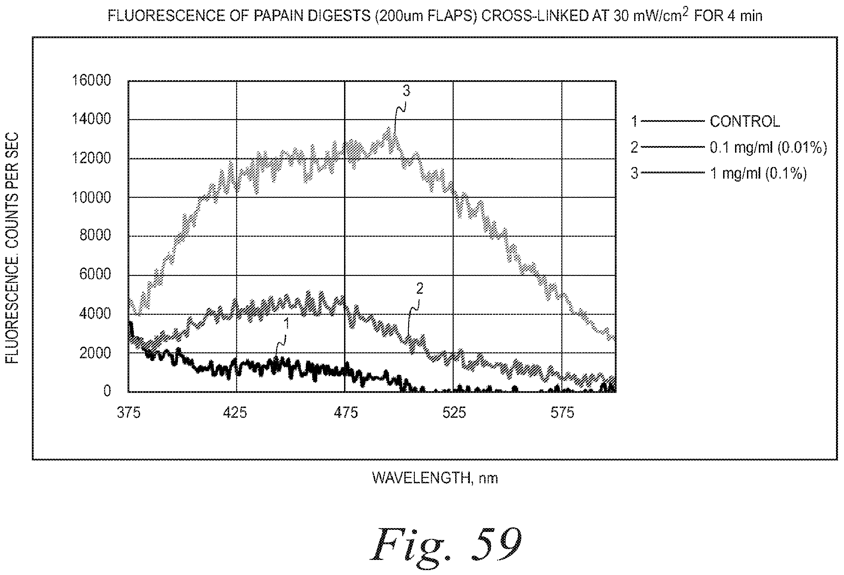

4-methyl-3-oxo-3,4-dihydro-2-quinoxaline carboxylic acid as shown in FIG. 56 has a strong absorbance at 360 nm (as shown in FIG. 57) and some fluorescence with a maximum around 460 nm (as shown in FIG. 58). FIG. 57 illustrates absorbance spectrum of 0.01 mg/ml solution of 4-methyl-3-oxo-3,4-dihydro-2-quinoxaline carboxylic acid in BBBS (quartz, 1 cm light path). FIG. 58 illustrates fluorescence of 4-methyl-3-oxo-3,4-dihydro-2-quinoxaline carboxylic acid in BBBS solutions (excitation 360 nm). FIG. 59 illustrates fluorescence of the papain-digested corneal flaps: non-cross-linked control (black line), cross-linked with 0.1 mg/ml (red line) and 1 mg/ml (green line) solutions of 4-methyl-3-oxo-3,4-dihydro-2-quinoxaline carboxylic acid in BBBS, where corneas were de-epithelialized, soaked with the solution of the cross-linker for 20 min and then irradiated for 4 min with 30 mW/cm$^2$ UVA light (360 nm). The compositions discussed in this paragraph do not fall within the scope of the invention.

Accordingly, the present disclosure provides systems and methods for use in treating the eye which employ cross-linking agents that are produced during the hydrolysis of riboflavin. For example, hydrolysis of riboflavin produces sodium salt of 1,2-Dihydro-6,7-dimethyl-2-keto-1-D-ribityl-3-quinoxalinecarboxylic acid monohydrate. It has been discovered that this salt is less susceptible to oxygen starvation (*i.e.,* lower sensitivity to oxygen presence) than conventional treatment solutions of riboflavin. When this salt is used alone or in combination with solutions of riboflavin, more effective cross-linking can be achieved for eye treatments.

Accordingly, the present disclosure provides systems and methods which employ thermal treatment of riboflavin solutions to achieve the results of the hydrolysis of riboflavin, including the production of cross-linking agents.

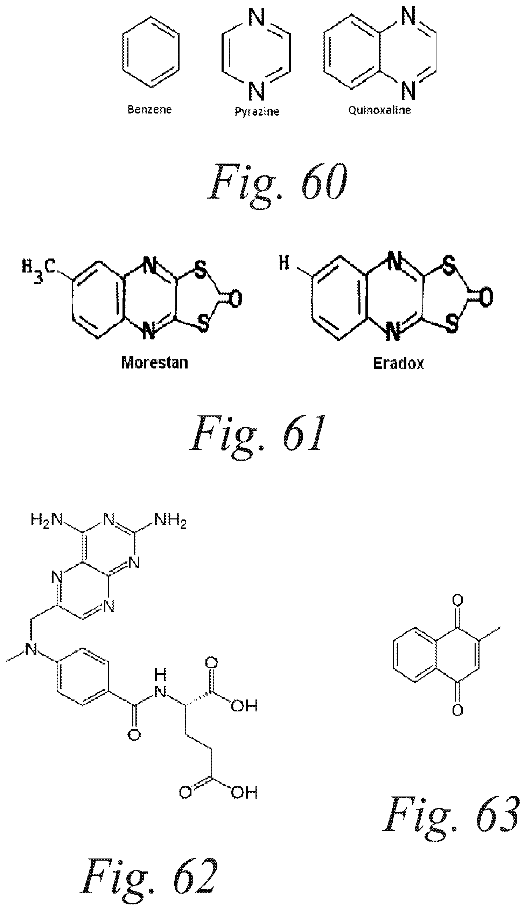

Accordingly, the present disclosure, provides systems and methods which employ quinoxalines to cause cross-linking activity for eye treatments. Quinoxaline (also called benzopyrazine) is a heterocyclic compound containing a ring complex made up of benzene ring and a pyrazine ring as shown in FIG. 60. Quinoxaline derivatives are widely distributed in nature and many of them, such as the antibiotics, echinomycin, levomycin and actinoleutin possess very useful biological activity. In addition, a large number of synthetic quinoxalines have also shown antibacterial, fungicidal, insecticidal, anticancer, tranquilizing and antidepressant properties. According to the present disclosure, the ability of the quinoxalines to act as cross-linking agents may be employed with these other benefits, e.g., antibacterial or fungicidal characteristics, for certain eye treatments. The collaborative work by synthetic and screening research groups have continuously been carried out to create various biologically active quinoxalines. Thus quinoxaline 1,4-dioxides have been shown antibacterial and quinoxaline-2,3-dithione cyclic dithio-carbonate (Morestan) and trithiocarbonate (Eradox) (as shown in FIG. 61) possess fungicidal and insecticidal effects. The 2,3,7-trichloro-6-methylsulfamoyl quinoxaline has been patented as anticancer agent. 2-Phenyl-3-piperidino quinoxaline and some of its derivatives are selective herbicides.

**Cross-linking with Olaquindox**

Olaquindox (N-(2-hydroxyethyl)-3-methyl-2-quinoxalinecarboxamide 1,4-dioxide) (VETRANAL™) has been used since 1975 as a growth promoter for farm animals because it has antibacterial characteristics. Olaquindox is related to the general class of quinoxalines. A commercially available preparation contains 10% olaquindox as an active ingredient in a calcium carbonate carrier together with 1.5% glyceryl polyethyleneglycol ricinoleate to reduce dust during preparation. The concentration of olaquindox in the feed is generally 50 ppm. Irradiated in the presence of human serum albumin, olaquindox disappears completely within 20 seconds; the N-monoxides are formed and a modified albumin which has altered properties in isoelectric focussing and electrophoresis systems. The following study evaluated the cross-linking potential of Olaquindox. The compositions used in this study do not fall within the scope of the invention.

A. Materials and Methods

Pig eyes were shipped overnight on ice from an abattoir (SiouxPreme, Sioux City, IA), rinsed in saline. Eyes were cleaned and epithelium was removed. Eyes were soaked for 20 minutes with 0.4% Olaquindox in PBS in an incubator set at 37°C by using a rubber ring to hold the solution on top. Some eyes were irradiated on air with continuous UVA light, and some placed in a beaker with a light oxygen stream for 2 minutes in the incubation chamber prior to irradiation. Corneas were pan-corneally irradiated with a top hat beam (3% root mean square) for 4 or 8 minutes with 365-nm light source (UV LED NCSU033B[T]; Nichia Co., Tokushima, Japan) at the chosen irradiance (30 mW/cm2, pulsed 1 sec on 1 sec off). UV irradiance was measured with a power sensor (model PD-300-UV; Ophir, Inc., Jerusalem, Israel) at the corneal surface. Corneal flaps (approximately 200 $\mu$m thick) were excised from the eyes with aid of Intralase femtosecond laser (Abbot Medical Optics, Santa Ana, CA). The average thickness of the corneal flaps was calculated as a difference between the measurements before and after the excision from the eyes with an ultrasonic Pachymeter (DGH Technology, Exton, PA. The flaps were placed into a biaxial extensometer (CellScale Biotester5000, Waterloo, ON), using biorake attachments with 5 tines spanning a width of 3 mm. Each sample was stretched at a constant rate of 4 $\mu$m/s in saline at 37°C until sample failure. The flaps were washed with distilled water 2 times, dried with filter paper, washed with dH$_2$O two times, and then dried in a vacuum until the weight change became less than 10% (Rotary vane vacuum pump RV3 A652-01-903, BOC Edwards, West Sussex, UK). Each flap was digested for 2.5 hours at 65°C with 2.5 units/ml of papain (from Papaya latex, Sigma) in 1 mL of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with 1XBBBS and fluorescence of the solutions was measured with excitation of $\lambda$ex = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

B. Results/Conclusion

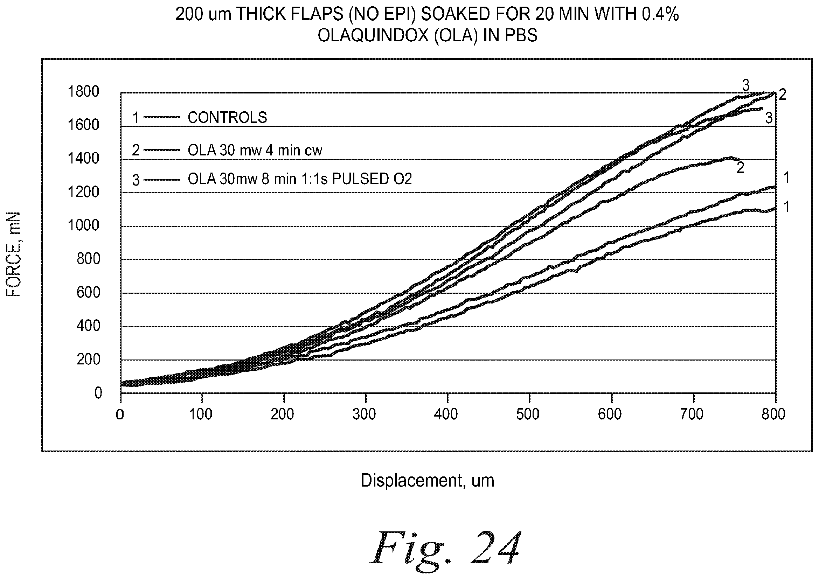

FIG. 24 illustrates biaxial extensiometry of 200 um thick corneal flaps, soaked with 0.4% Olaquindox in PBS with irradiation for 4 min with 30 mW/cm2 (CW) (2) and irradiation for 8 minutes (1 second on:1 second off) with 30 mW/cm2 of pulsed UVA light and O$_2$ (3), relative to controls (1).

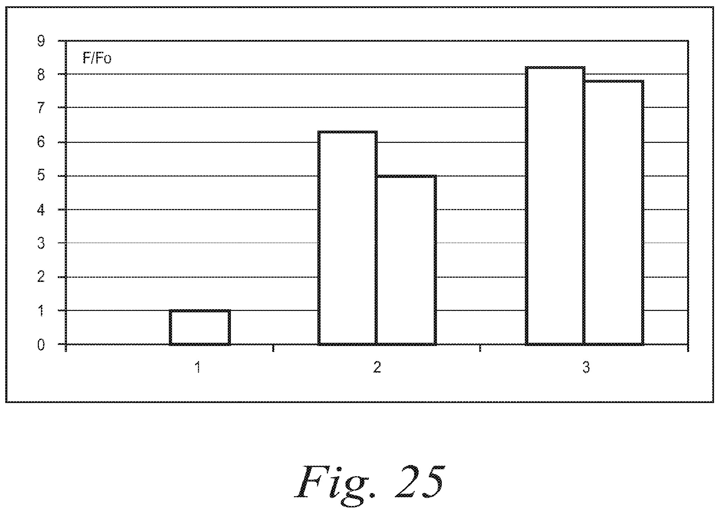

FIG. 25 illustrates relative fluorescence recorded at 450 nm of the cross-linked flaps with 0.4% Olaquindox in PBS: (1) non-irradiated control; (2) irradiation for 4 min with 30 mW/cm$^2$ continuously (CW); and (3): irradiation for 8 minutes (1 second on:1 second off) with 30 mW/cm2 of pulsed UVA light and O$_2$.

After exposure with UVA, corneal collagen became stiffer and acquired fluorescence at 450 nm. As such, Olaquindox is an effective water-soluble cross-linking agent.

**Cross-linking with Riboflavin and Folic Acid**

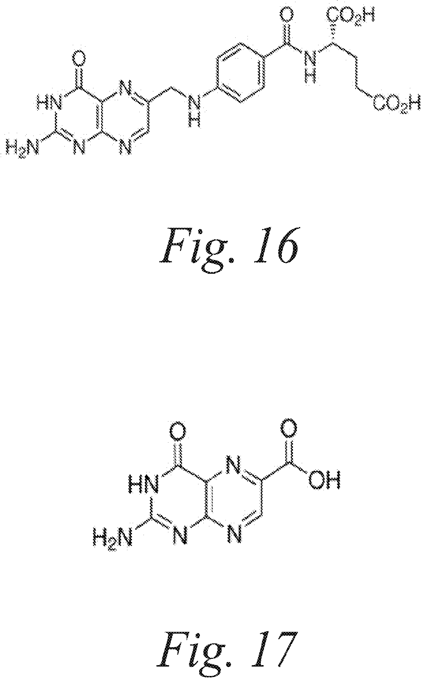

Folic acid (FA), or vitamin B$_9$, as shown in FIG. 16 is specifically considered by the United States Food and Drug Administration to be among safe medical food ingredients, under 21 CFR § 172.345(f). FA itself does not have a strong fluorescence and does not sensitize formation of singlet oxygen efficiently (most probably due to involvement of the p-aminobenzoylglutamate substituent in internal fluorescence quenching by radiationless deactivation of the singlet excited state leading to inefficient intersystem crossing). However, UV irradiation causes the oxidation of FA, leading to the formation of 6-formylpterin and then pterine-6-carboxylic acid (PCA) as shown in FIG. 17.

PCA is an efficient photosensitizer and generator of singlet oxygen. Therefore, both FA and PCA can generate collagen cross-linking. FA may be used in combination with riboflavin because addition of riboflavin markedly intensifies oxidation of FA while most of the riboflavin remains undecomposed.

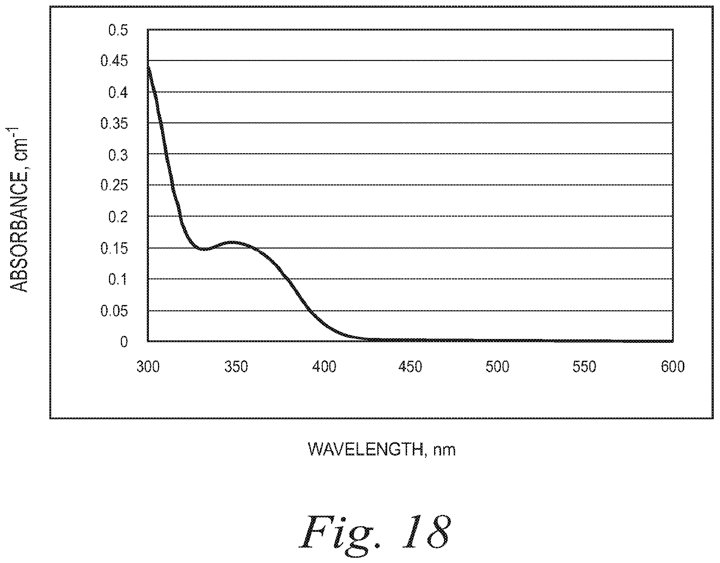

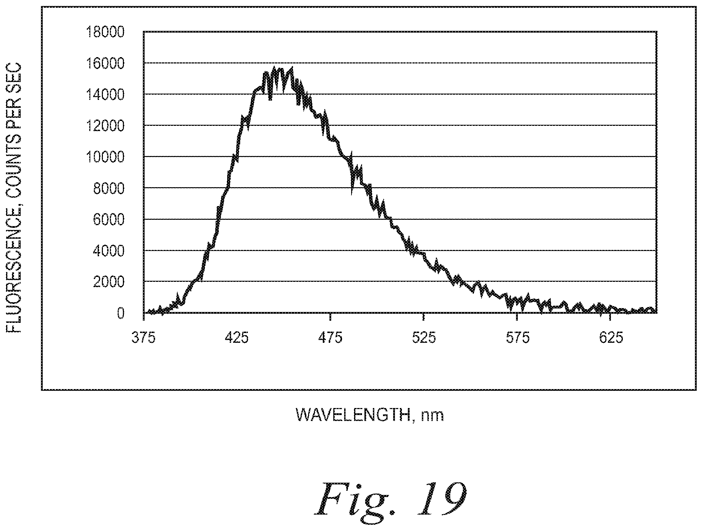

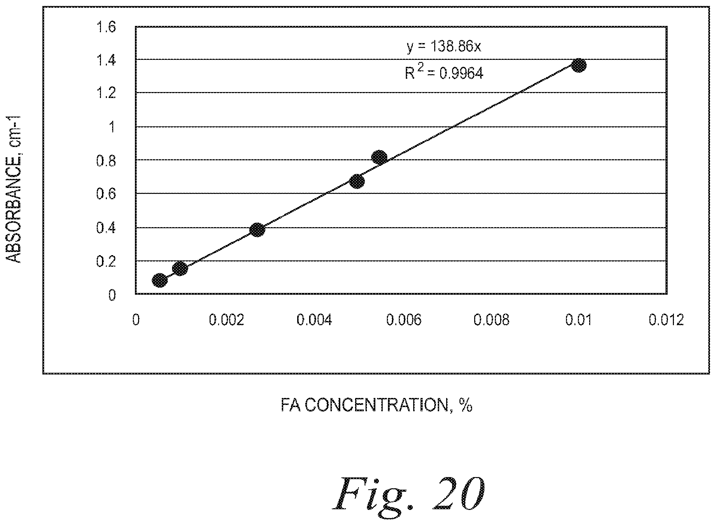

FA is soluble in water with dependence on pH and temperature. In the following studies, for example, its solubility in a phosphate buffer was 5.5 mg/ml at 25°C and pH of the final solution was 7.0. FA has UV light absorbance at 400 nm and below (the long wave peak at 360 nm as shown in FIG. 18), and fluorescence with maximum around 460 nm (as shown in FIG 19). FIG. 20 shows the absorbance of FA at 360 nm as a function of FA concentration in phosphate buffer. The compositions used in this study do not fall within the scope of the invention.

A. Materials and Methods

Pig eyes were shipped overnight on ice from an abattoir (SiouxPreme, Sioux City, IA), rinsed in saline. The eyes were cleaned and epithelium was removed. Sodium Phosphate Buffer (pH 7.6, made with Sodium Phosphate Monobasic, Sodium Phosphate Dibasic and Sodium Chloride in distilled water) was used as the buffer for all solutions. The final pH values for riboflavin solution, FA solution, and their mixture were in the range of 7.3-7.4. The eyes were soaked for 20 minutes with 0.1% FA, 0.1% Riboflavin, or 0.1% FA + 0.1% riboflavin in an incubator set at 37°C by using a rubber ring to hold the solution on top. The eyes were placed in a beaker filled with pure oxygen for 2 minutes in the incubation chamber prior to irradiation. Corneas were pan-corneally irradiated with a top hat beam (3% root mean square) for 8 minutes with 365-nm light source (UV LED NCSU033B[T]; Nichia Co., Tokushima, Japan) at the chosen irradiance (30 mW/cm$^2$, pulsed 1 second on: 1 second off) which was measured with a power sensor (model PD-300-UV; Ophir, Inc., Jerusalem, Israel) at the corneal surface. Corneal flaps (approximately 200 $\mu$m thick) were excised from the eyes with aid of Intralase femtosecond laser (Abbot Medical Optics, Santa Ana, CA). The average thickness of the corneal flaps was calculated as a difference between the measurements before and after the excision from the eyes with an ultrasonic Pachymeter (DGH Technology, Exton, PA). The flaps were placed into a biaxial extensometer (CellScale Biotester 5000, Waterloo, ON), using biorake attachments with 5 tines spanning a width of 3 mm. Each sample was stretched at a constant rate of 4 $\mu$m/s in saline at 37°C until sample failure. The flaps were washed with distilled water 2 times, dried with filter paper, washed with dH$_2$O two times, and then dried in a vacuum until the weight change became less than 10% (Rotary vane vacuum pump RV3 A652-01-903, BOC Edwards, West Sussex, UK). Each flap was digested for 2.5 hours at 65°C with 2.5 units/ml of papain (from Papaya latex, Sigma) in 1 ml of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with 1XBBBS and fluorescence of the solutions was measured with excitation of $\lambda$ex = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

B. Results/Conclusion

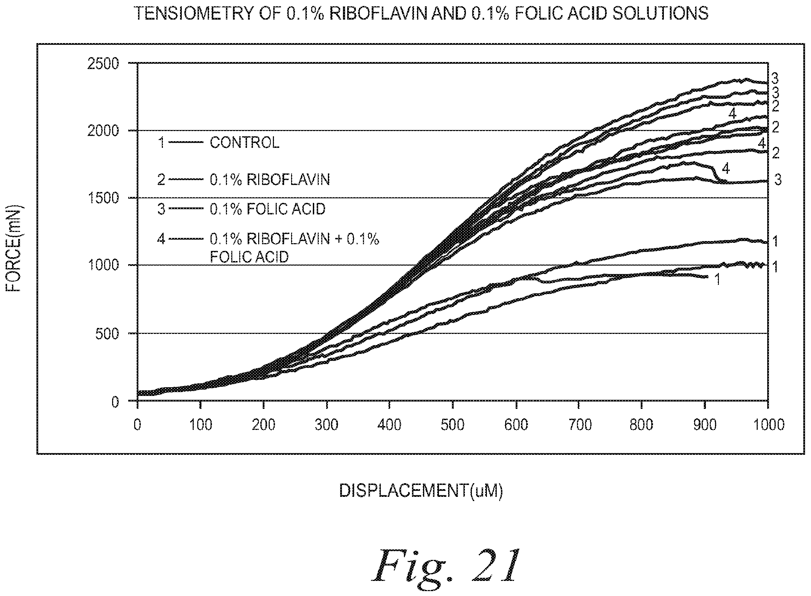

FIG. 21 illustrates displacement vs. force curves for corneal samples: (1) not exposed to UV light; (2) 0.1% riboflavin, exposed to UV light; (3) 0.1% FA, exposed to UV light; and (4) mixture of 0.1% riboflavin and 0.1% FA, exposed to UV light, with UV exposure of 365 nm, 30 mW/cm$^2$, pulsed 1 second on: 1 second off for 8 minutes total, with oxygen ambience over the cornea.

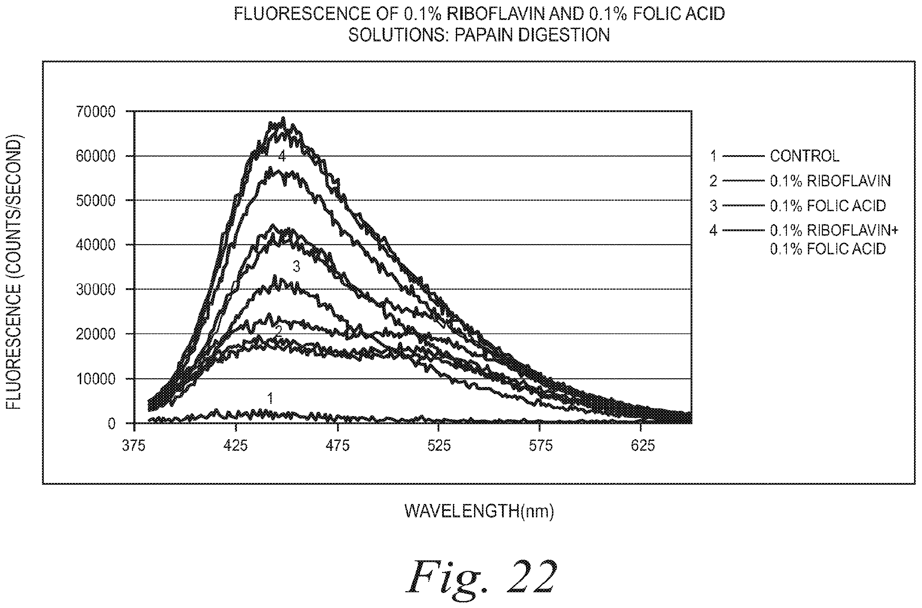

FIG. 22 illustrates fluorescence of the corneal samples after digestion with papain (excitation 360 nm): (1) not exposed to UV light; (2) 0.1% riboflavin, exposed to UV light; (3) 0.1% FA, exposed to UV light; and (4) mixture of 0.1% riboflavin and 0.1% FA, exposed to UV light, with UV exposure at 365 nm, 30 mW/cm$^2$, pulsed 1 second on: 1 second off for 8 minutes total, with oxygen ambience over the cornea.

As shown in FIG. 21, the effect of the exposure of UV light on corneal collagen is very similar for all three groups of the studied solutions (0.1% riboflavin, 0.1% FA, and mixture of 0.1% riboflavin with 0.1% FA). As compared to the unexposed controls, soaking with a solution and then exposing it to UV leads to significant stiffening of a corneal sample. FIG. 22 shows that fluorescence of the cross-linked collagen samples increases as compared to the non-exposed to UV control samples.

As described above, it is also contemplated that FA can also be used as a primary cross-linking agent (without riboflavin). The compositions used in this study do not fall within the scope of the invention.

C. Additional Experiment

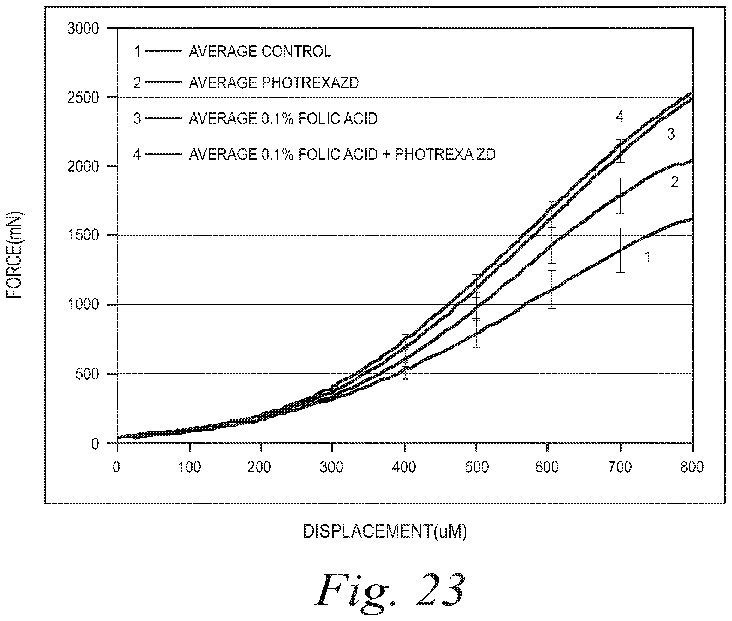

An additional experiment was conducted when FA was dissolved in a formulation solution containing 0.1% riboflavin in buffer saline solution (available under AVEDRO® PHOTREXA ZD™). FIG. 23 illustrates displacement vs. force curves for corneal samples (thickness 300 um, 3 samples in each group): (1) controls unexposed to UV light; (2) 0.1% FA in a buffer, exposed UV light; (3) 0.1% riboflavin in buffer saline solution, exposed to UV light; and (4) mixture of 0.1% FA in 0.1% riboflavin in buffer saline solution, exposed to UV light, UV exposure at 365 nm, 30 mW/cm$^2$, pulsed 1 second on: 1 second off for 8 minutes total, with oxygen ambience over the cornea.

## Other Cross-Linking Agents

The drugs chloroquine, hydroxychloroquine, quinine, and dibucaine may possess photosensitizing capability in aqueous solutions, e.g., by irradiation with 365 nm UV light. Chloroquine, hydroxychloroquine, and quinine are related to the general class of quinolines. As described in the present disclosure, these drugs, based on their photosensitizing capability, may be applied as cross-linking agents in treatments of the cornea.

Methotrexate, as shown in FIG. 62, is a generic name of an immunosuppressive medication which has been used for treatment of certain cancers, and inflammatory diseases such as rheumatoid arthritis and uveitis. Similarly to FA, MTX has a significant UV-light absorbance at 360 nm. An idea of using MTX as a possible collagen cross-linker comes from the data about MTX photosensitizing properties measured on rabbit eyes conjunctive. This does not fall within the scope of the present invention.

Pronounced UVA-photosensitization of thymidine has been observed with menadione (vitamin K$_3$), as shown in FIG. 63, and formation of the photo-oxidation product 5,6-dihydroxy-5,6-dihydrothymidine occurs after single electron transfer reactions between triplet state menadione and thymidine. Menadione can be a UVA photosensitizer, and therefore, a cross-linking agent for collagen. This does not fall within the scope of the present invention.

Accordingly, various agents and additives for cross-linking treatments are identified and described in studies. The characteristics of the various agents and additives may be advantageously employed in formulations applied in cross-linking treatments of the eye. In some disclosures, riboflavin is combined with Iron(II) to enhance the cross-linking activity generated by the riboflavin. In other disclosures, cross-linking treatments employ an Iron(II) solution in combination with a hydrogen peroxide pre-soak. In yet other disclosures, 2,3-butanedione is employed to increase the efficacy of corneal cross-linking with a photosensitizer, such as riboflavin. In further disclosures, folic acid is employed in combination with a photosensitizer, such as riboflavin, to enhance cross-linking activity. In yet further disclosures, 2,3-butanedione, folic acid, a quinoxaline, a quinoline, or dibucaine is applied as a cross-linking agent.

As described above, according to some aspects of the present disclosure, some or all of the steps of the above-described and illustrated procedures can be automated or guided under the control of a controller (e.g., the controller 120). Generally, the controllers may be implemented as a combination of hardware and software elements. The hardware aspects may include combinations of operatively coupled hardware components including microprocessors, logical circuitry, communication/networking ports, digital filters, memory, or logical circuitry. The controller may be adapted to perform operations specified by a computer-executable code, which may be stored on a computer readable medium.

As described above, the controller may be a programmable processing device, such as an external conventional computer or an on-board field programmable gate array (FPGA) or digital signal processor (DSP), that executes software, or stored instructions. In general, physical processors and/or machines employed for any processing or evaluation may include one or more networked or non-networked general purpose computer systems, microprocessors, field programmable gate arrays (FPGA's), digital signal processors (DSP's), micro-controllers, and the like, programmed according to the teachings of the present disclosure, as is appreciated by those skilled in the computer and software arts. The physical processors and/or machines may be externally networked with the image capture device(s), or may be integrated to reside within the image capture device. Appropriate software can be readily prepared by programmers of ordinary skill based on the present disclosures, as is appreciated by those skilled in the software art. In addition, the devices and subsystems described herein can be implemented by the preparation of application-specific integrated circuits or by interconnecting an appropriate network of conventional component circuits, as is appreciated by those skilled in the electrical art(s). Thus, the example embodiments are not limited to any specific combination of hardware circuitry and/or software.

Stored on any one or on a combination of computer readable media, the present disclosures may include software for controlling the devices and subsystems described herein, for driving the devices and subsystems described herein, for enabling the devices and subsystems described herein to interact with a human user, and the like. Such software can include, but is not limited to, device drivers, firmware, operating systems, development tools, applications software, and the like. Such computer readable media further can include the computer program product t of the present disclosure for performing all or a portion (if processing is distributed) of the processing performed in implementations. Computer code devices of the present disclosure can include any suitable interpretable or executable code mechanism, including but not limited to scripts, interpretable programs, dynamic link libraries (DLLs), Java classes and applets, complete executable programs, and the like. Moreover, parts of the processing of the present disclosure can be distributed for better performance, reliability, cost, and the like.

Common forms of computer-readable media may include, for example, a floppy disk, a flexible disk, hard disk, magnetic tape, any other suitable magnetic medium, a CD-ROM, CDRW, DVD, any other suitable optical medium, punch cards, paper tape, optical mark sheets, any other suitable physical medium with patterns of holes or other optically recognizable indicia, a RAM, a PROM, an EPROM, a FLASH-EPROM, any other suitable memory chip or cartridge, a carrier wave or any other suitable medium from which a computer can read.

While the present disclosure has been described with reference to one or more particular embodiments, those skilled in the art will recognize that many changes may be made. It is also contemplated that additional embodiments according to aspects of the present disclosure may combine any number of features from any of the embodiments described herein.

**Claims**

1.   A composition for use in a method for applying treatment to a cornea of an eye, the method comprising:

applying the composition to the cornea, the composition including:

a cross-linking agent including at least one of riboflavin, 2,3-butanedione, folic acid, quinolone, dibucaine, or a quinoxaline;
an iron additive; and
citrate buffer, and

applying photo-activating light to the cornea to generate cross-linking activity in the cornea.

2.   The composition for the use according to claim 1, wherein the iron additive includes $FeSO_4$.

3.   The composition for the use according to claim 1 or claim 2, wherein the iron additive is dissolved in the citrate buffer.

4.   The composition for the use according to any one of claims 1 to 3, wherein the photo-activating light is ultraviolet light.

5.   The composition for the use according to any one of claims 1 to 4, wherein the photo-activating light is pulsed.

6.   The composition for the use according to any one of claims 1 to 5, wherein the photo-activating light is continuous.

7.   The composition for the use according to any one of claims 1 to 6, further comprising applying a selected concentration of oxygen to the eye, the selected concentration being greater than a concentration of oxygen in atmosphere.

8.   The composition for the use according to any one of claims 1 to 7, wherein the cornea is weakened due to keratoconus.

9.   The composition for the use according to any one of claims 1 to 7, wherein the cornea is weakened due to Laser-Assisted in situ Keratomileusis (LASIK) ectasia.

**Patentansprüche**

1.   Zusammensetzung zur Verwendung in einem Verfahren zum Anwenden einer Behandlung auf die Hornhaut eines Auges, wobei das Verfahren Folgendes umfasst:

das Anwenden der Zusammensetzung auf die Hornhaut, wobei die Zusammensetzung Folgendes umfasst:

ein Vernetzungsmittel, das zumindest eines aus Riboflavin, 2,3-Butandion, Folsäure, Chinolon, Dibucain und Chinoxalin umfasst,
ein Eisenadditiv und
Citratpuffer; und

das Anwenden von photoaktivierendem Licht auf die Hornhaut, um in der Hornhaut eine Vernetzungsaktivität zu erzeugen.

2.   Zusammensetzung zur Verwendung nach Anspruch 1, wobei das Eisenadditiv $FeSO_4$ umfasst.

**3.** Zusammensetzung zur Verwendung nach Anspruch 1 oder Anspruch 2, wobei das Eisenadditiv in dem Citratpuffer gelöst ist.

**4.** Zusammensetzung zur Verwendung nach einem der Ansprüche 1 bis 3, wobei das photoaktivierende Licht UV-Licht ist.

**5.** Zusammensetzung zur Verwendung nach einem der Ansprüche 1 bis 4, wobei das photoaktivierende Licht gepulst wird.

**6.** Zusammensetzung zur Verwendung nach einem der Ansprüche 1 bis 5, wobei das photoaktivierende Licht kontinuierlich ist.

**7.** Zusammensetzung zur Verwendung nach einem der Ansprüche 1 bis 6, die weiters das Anwenden einer ausgewählten Konzentration von Sauerstoff auf das Auge umfasst, wobei die ausgewählte Konzentration höher ist als die Konzentration von Sauerstoff in der Atmosphäre.

**8.** Zusammensetzung zur Verwendung nach einem der Ansprüche 1 bis 7, wobei die Hornhaut aufgrund eines Keratokonus geschwächt ist.

**9.** Zusammensetzung zur Verwendung nach einem der Ansprüche 1 bis 7, wobei die Hornhaut aufgrund einer laserunterstützten In-situ-Keratomileusis- (LASIK-) Ektasie geschwächt ist.

## Revendications

**1.** Composition pour utilisation dans un procédé pour appliquer le traitement à une cornée d'un œil, le procédé comprenant les étapes consistant à :
appliquer la composition sur la cornée, la composition incluant :

un agent de réticulation incluant au moins un parmi la riboflavine, le 2,3-butanedione, l'acide folique, la quinolone, la dibucaïne, ou une quinoxaline ;
un additif de fer ; et
un tampon de citrate, et
appliquer une lumière photo-activante à la cornée pour générer une activité de réticulation dans la cornée.

**2.** Composition pour utilisation selon la revendication 1, dans laquelle l'additif de fer comprend du $FeSO_4$.

**3.** Composition pour utilisation selon la revendication 1, dans laquelle l'additif de fer est dissous dans le tampon de citrate.

**4.** Composition pour utilisation selon l'une quelconque des revendications 1 à 3, dans laquelle la lumière photo-activante est la lumière ultraviolette.

**5.** Composition pour utilisation selon l'une quelconque des revendications 1 à 4, dans laquelle la lumière photo-activante est pulsée.

**6.** Composition pour utilisation selon l'une quelconque des revendications 1 à 5, dans laquelle la lumière photo-activante est continue.

**7.** Composition pour utilisation selon l'une quelconque des revendications 1 à 6, comprenant en outre l'application d'une concentration sélectionnée d'oxygène à l'œil, la concentration sélectionnée étant supérieure à une concentration d'oxygène dans l'atmosphère.

**8.** Composition pour l'utilisation selon l'une quelconque des revendications 1 à 7, dans laquelle la cornée est affaiblie par un kératocône.

**9.** Composition pour utilisation selon l'une quelconque des revendications 1 à 7, dans laquelle la cornée est affaiblie en raison d'une ectasie de kératomileusis *in situ* assisté par laser (LASIK).

*Fig. 1*

*Fig. 2A*

*Fig. 2B*

*Fig. 3*

hydrogen dioxide

$$HO_2^{\bullet}$$

$$O_2 \xrightarrow{+\,1\,e^-} O_2^{\bullet -} \xrightarrow[\text{or} +O_2^{\bullet -}]{+\,1\,e^-} H_2O_2 \xrightarrow{+\,1\,e^-} HO^{\bullet} \longrightarrow H_2O$$

| Molecular oxygen | superoxide anion | hydrogen peroxide | hydroxyl radical | water |

$$RH + HO^{\bullet} \rightarrow R^{\bullet} + H_2O$$

$$RH + HO_2^{\bullet} \rightarrow R^{\bullet} + H_2O_2$$

$$RH + {}^1O_2 \longrightarrow ROOH$$

*Fig. 4*

*Fig. 5*

*Fig. 6*

EP 4 082 547 B1

RELATIVE FLUORESCENCE AT 450nm

Fig. 7

0.1 % Ribo, 0.5mMFeSO4, CW

Fig. 8

EP 4 082 547 B1

0.1% Ribo, 0.5mMFeSO4 PULSED+O2

1 - CONTROL

2- 0.1% RIBOFLAVIN

3 - 0.1% RIBOFLAVIN + 0.5mM FeSO4

DISPLACEMENT, uM

*Fig. 9*

0.1% Ribo, 0.5mMFeSO4 PULSED+O2, REPEAT

Fig. 10

Riboflavin

Endoperoxide

Dioxetane

2, 3-Butanedione

R = Ribose

*Fig. 11*

*Fig. 12*

*Fig. 13*

*Fig. 14*

EP 4 082 547 B1

*Fig. 15*

## Fig. 16

## Fig. 17

EP 4 082 547 B1

Fig. 18

*Fig. 19*

Fig. 20

EP 4 082 547 B1

TENSIOMETRY OF 0.1% RIBOFLAVIN AND 0.1% FOLIC ACID SOLUTIONS

*Fig. 21*

FLUORESCENCE OF 0.1% RIBOFLAVIN AND 0.1% FOLIC ACID
SOLUTIONS: PAPAIN DIGESTION

1 —— CONTROL
2 —— 0.1% RIBOFLAVIN
3 —— 0.1% FOLIC ACID
4 —— 0.1% RIBOFLAVIN+ 0.1% FOLIC ACID

*Fig. 22*

EP 4 082 547 B1

*Fig. 23*

EP 4 082 547 B1

200 um THICK FLAPS (NO EPI) SOAKED FOR 20 MIN WITH 0.4%
OLAQUINDOX (OLA) IN PBS

1 ——— CONTROLS

2 ——— OLA 30 mw 4 min cw

3 ——— OLA 30mw 8 min 1:1s PULSED O2

FORCE, mN

Displacement, um

*Fig. 24*

*Fig. 25*

*Fig. 26*

*Fig. 27*

*Fig. 28*

*Fig. 29*

*Fig. 30*

Fig. 31

**1**
$C_{17}H_{20}N_4O_6$
Mol. Wt.: 376,36

**2**
$C_{16}H_{21}N_2NaO_8$
Mol. Wt.: 392,34

*Fig. 32*

Sodium salt of 1,2-Dihydro-6,7-dimethyl-2-keto-1-D-ribityl-3-quinoxalinecarboxylic
Acid Monohydrate
prod. no. 5082; lot. no. 20130301
1H-NMR in DMSO-d6 (26-03-13)

$C_{16} H_{19} N_2 NaO_8 \times H_2 O$
Mol. Wt: 374.32 + $H_2 O$

*Fig. 33*

RIBOFLAVIN 5 PHOSPH AUF WP300 30MIN ISO15PROZMEOH KH2PO4_40GRAD #3

*Fig. 34*

5mg FMN+10mg Tris/ml 0,85% NaCl/5 1h 115°C    UV_VIS_2

*Fig. 35*

RIBOFLAVIN 5 PHOSPH AUF WP300 30MIN ISO15PROZMEOH KH2PO4_40GRAD #7

*Fig. 36*

RIBOFLAVIN 5 PHOSPH AUF WP300 30MIN ISO15PROZMEOH KH2PO4_40GRAD #8

*Fig. 37*

*Fig. 38*

*Fig. 39*

*Fig. 40*

*Fig. 41*

*Fig. 42*

*Fig. 43*

**Fig. 44**

*Fig. 45*

EP 4 082 547 B1

ABSORBANCE AT 360 nm (1 cm, BBBS, QUARTZ)

*Fig. 46*

EXCITATION 360nm

1 —— 0_0001%
2 —— 0_001%
3 —— 0_002%
4 —— 0_005%

FLUORESCENCE, COUNTS PER SEC

120000
100000
80000
60000
40000
20000
0

375  425  475  525  575  625

WAVELENGTH, nm

*Fig. 47*

*Fig. 48*

*Fig. 49*

*Fig. 50*

Fig. 51

*Fig. 52*

Fig. 53

*Fig. 54*

*Fig. 55*

*Fig. 56*

*Fig. 57*

Fig. 58

FLUORESCENCE OF PAPAIN DIGESTS (200um FLAPS) CROSS-LINKED AT 30 mW/cm$^2$ FOR 4 min

1 ——— CONTROL
2 ——— 0.1 mg/ml (0.01%)
3 ——— 1 mg/ml (0.1%)

WAVELENGTH, nm

*Fig. 59*

EP 4 082 547 B1

Benzene    Pyrazine    Quinoxaline

## Fig. 60

Morestan    Eradox

## Fig. 61

## Fig. 62

## Fig. 63

PHOTREXA ZD VS. PHOTREXA ZD +0.25mM FeSO4

*Fig. 64*

PHOTREXA ZD VS. PHOTREXA ZD +0.5mM FeSO4

PHOTREXA ZD, PW+O2

PHOTREXA ZD + 0.5mM
FeSO4 AND 0.25mM CITRATE

*Fig. 65*

EP 4 082 547 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62086572
- US 20140343480 A1, KAMAEV PAVEL
- US 2013310732 A1, FOSCHINI FULVIO
- US 20110237999
- US 20120215155
- US 20130245536
- US 8574277 B
- US 20130060187
- US 1557628 W
- US 62255452

**Non-patent literature cited in the description**

- **SURREY et al.** *J. Am. Chem. Soc.*, 1951, vol. 73, 3236-2338